# EUROPEAN PATENT SPECIFICATION

(11) **EP 1 849 395 B1**
(45) Date of publication and mention of the grant of the patent: **30.03.2011**
(21) Application number: 06712071.7
(22) Date of filing: 20.01.2006
(51) Int. Cl.: A61B 1/04, A61B 5/07, A61B 5/04

(54) **PORTABLE MEDICAL APPARATUS STORAGE HOLDER AND CAPSULE ENDOSCOPE MEDICAL CARE SYSTEM**
AUFNAHMEHBEHAELTER FÜR TRAGBARE MEDIZINISCHE BEHANDLUNGSVORRICHTUNG UND MEDIZINISCHES BEHANDLUNGSSYSTEM MIT KAPSELENDOSKOP
SUPPORT DE MAINTIEN POUR APPAREIL PORTABLE DE TRAITEMENT MEDICAL ET SYSTEME DE TRAITEMENT MEDICAL ENDOSCOPIQUE DE TYPE CAPSULE

(30) Priority: 17.02.2005 JP 2005040948
(43) Date of publication of application: 31.10.2007
(73) Proprietor: OLYMPUS MEDICAL SYSTEMS CORP., Shibuya-ku Tokyo 151-0072 (JP)
(72) Inventor: AKAGI Toshimasa, Tokyo 151-0072 (JP); SEGAWA Hidetake, Tokyo 151-0072 (JP)
(74) Representative: von Hellfeld, Axel
(86) International application number: PCT/JP2006/300846
(87) International publication number: WO 2006/087886

(56) References cited:
- EP-A- 1 342 447
- WO-A-01/26232
- WO-A-2005/004033
- JP-A- 62 015 897
- JP-A- 2002 263 075
- JP-A- 2003 135 389
- US-A1- 2002 188 216
- US-A1- 2003 023 150

## Description

### TECHNICAL FIELD

The present invention relates to a portable medical apparatus storage holder and a capsule endoscope medical care system.

### BACKGROUND ART

Conventionally, there is known a capsule endoscope (swallowing type tablet-shaped endoscope for medical care) which can be taken from a mouth into a body cavity to collect information in the body cavity of a living body by imaging digestive organs such as stomach. There is proposed a capsule endoscope including an illumination unit formed by LED or the like, a solid-state imaging element formed by CCD or CMOS, a transmitting unit for transmitting image data obtained by the solid-state imaging element to the outside, and a power supply unit having a battery for driving the illumination unit, the solid-state imaging element, and the transmitting unit. All the components are incorporated in a capsule.

During medical care with the capsule endoscope, a radio wave transmitted from the capsule swallowed by a subject is caught by loop antennas at plural points on a body surface of the subject, e.g., by the loop antennas adhering to eight points, and the caught data is transmitted to a receiver through an antenna cable to record the data in a CompactFlash® memory of the receiver. Although eight to ten hours are required until the medical care is finished, the subject can have an ordinary life while putting on the loop antenna and the receiver during the medical care. When the measurement with the capsule endoscope finishes, the subject submits the receiver to a hospital. On the hospital side, the receiver is inserted into a cradle and a workstation connected to the cradle captures all the pieces of measurement data recorded in the receiver through a USB cable. Then, the measurement result is observed as a moving picture with the workstation.

Thus, because the subject puts on the receiver as long as eight to ten hours, a receiver holder for retaining the receiver is required so that the subject has no trouble in the ordinary life.

There are conventionally proposed many holders for various kinds of portable instruments. The receiver holder differs largely from general holders such as a baggage, a waist pouch, and a portable holder in that a storage target of the receiver holder is a receiver which is electrically connected by the loop antenna and antenna cable adhering to the subject body.

On the other hand, for example, in an electrocardiograph, there has been proposed a holder which retains an electrocardiogram signal processing device on the subject body, and the electrocardiogram signal processing device on the outside of the cloth is connected to electrodes adhering to a patient through a cable (for example, see Patent Documents 1 to 3).

Patent Document 1: Japanese Patent Application Laid-Open No. H5-220119
Patent Document 2: Japanese Patent Application Laid-Open No. 2003-220043
Patent Document 3: Japanese Patent Application Laid-Open No. 2004-261282

Document WO 01/26232 A2 describes a portable EEG monitoring device having a patient worn amplifier and a portable operations device connected to the amplifier. A jackbox has a plurality of electrode connectors. The jackbox is connected to the amplifier by a cable, whereas the cable is preferably routed between the jackbox and the amplifier in a recess or a groove in one or both devices. The amplifier is held within a holster with at a least one opening. The holster is secured to the patient via a belt passed through one or more loop holes in the holster. The amplifier is mounted upon the portable operations device, whereas the portable operations device is also received within the holster.

Document WO 2005/004033 A2 describes an in-vivo imaging system for receiving and recording signals from a body cavity or lumen. To this end, antennas/sensors are attached to the skin of a user. The sensors are connected to a receiver, which in turn is connected to a recorder via a connecting cable. The system is incorporated within a wearable article of clothing such as a belt with pockets.

### DISCLOSURE OF INVENTION

### PROBLEM TO BE SOLVED BY THE INVENTION

However, in the holder disclosed in Patent Documents 1 to 3, behavior of the cable exposed in a bared state in the vicinity of the holder becomes unstable. Particularly, in the case of the above-described receiver holder, the antenna cable of the loop antenna adhering to a bare skin of the subject is drawn to the outside of the cloths and connected to the receiver while slipping through the cloths such as shirt, underpants, and trousers. Therefore, when the cable whose behavior is unstable in the vicinity of the holder in which the receiver is stored is moved violently or fluctuates by some sort of cause, sometimes there is generated a trouble such that accidental external force is applied to the cable and position of the loop antenna is shifted. The cable has a general property in which a core wire is easily disconnected due to metal fatigue caused by repeated bending deformation. Therefore due to the unstable behavior of the cable, sometimes the cable is disconnected at a base end portion by steeply bending the base end portion of the cable drawn from the holder.

The present invention has been made in view of the circumstance. It is an object to provide a portable medical apparatus storage holder and a capsule endoscope medical care system, wherein unstable behavior of the drawn cable connected to the portable medical apparatus which is of the storage target can be eliminated to prevent cable jam or looseness and cable disconnection.

### MEANS FOR SOLVING PROBLEM

A portable medical apparatus storage holder according to one aspect of the present invention includes a storage container in which a portable medical apparatus is detachably stored from an opening, the portable medical apparatus being placed on a subject body, the portable medical apparatus being electrically connected to a detecting device through a cable to record a predetermined electric displacement amount detected by the detecting device detecting; a placing member which detachably places the storage container on the subject body; and a constraint member which is continuously connected to the storage container or the placing member, the constraint member constraining a path of the cable drawn from the opening of the storage container in which the portable medical apparatus is stored.

In the portable medical apparatus storage holder, the opening is an opening which is positioned so as to face the subject side while the storage container is placed, the opening permitting the portable medical apparatus to be inserted and detached, the opening permitting the cable to be drawn, the opening includes a cap member which is continuously connected to the storage container at a side on a top portion side of the opening by a hinge pair to open and close the opening, the cap member having a size in which an end side parallel to the hinge pair overlaps an outer surface on a backside of the storage container when the opening is closed; and a joint structure which joints an inner surface of the cap member and the outer surface on the backside of the storage container in an overlapping range between the cap member and the storage container, the constraint member has the joint structure, the cable includes a plurality of cables, and the constraint member constrains a path of a base portion of the cables by clamping a bundling member with the joint structure portion, the bundling member bundling the cables in a flat shape, the cables being drawn to the subject side from the opening of the storage container in which the portable medical apparatus is stored.

In the portable medical apparatus storage holder, the joint structure may be a joint structure in which a surface fastener is used.

In the portable medical apparatus storage holder, the storage container may have a groove structure in which the cable and the bundling member are fitted in a vertical direction in the joint structure.

In the portable medical apparatus storage holder, the constraint member may include a cushioning member arranged at a position included in an inside of the bent cable in a region where a base portion of the cable is bent on a path through which the cable is drawn from the storage container through the opening.

In the portable medical apparatus storage holder, the placing member may include a suspension placing member which is suspended on a shoulder of the subject to place the storage container on a middle abdomen portion of the subject, and the constraint member may include a pulling member which is provided in the suspension placing member to pull a neighbor of the base portion of the cable drawn from the opening.

In the portable medical apparatus storage holder, the constraint member may include a strap member which routes the cable drawn from the opening to surround the cable on the outer surface side of the storage container.

In the portable medical apparatus storage holder, the storage container may include a friction member in a backside thereof which is in contact with the subject body, the friction member having elasticity.

In the portable medical apparatus storage holder, the storage container and the cap member may include friction members in backsides thereof which are in contact with the subject body, the friction member having elasticity.

In the portable medical apparatus storage holder, the placing member may include an abdominal region placing member and a suspension placing member, the abdominal region placing member being rounded around a abdomen portion of the subject, the suspension placing member being suspended on the shoulder of the subject while detachably attached at an arbitrary position of the abdominal region placing member.

In the portable medical apparatus storage holder, the placing member may be freely adjustable in length according to a habitus of the subject.

In the portable medical apparatus storage holder, the placing member may be formed by a disposable material which can freely be cut with a cutting tool, and the placing member may be freely adjustable in length by cutting the placing member according to the habitus of the subject.

In the portable medical apparatus storage holder, the storage container may be formed by disposable nonwoven cloth, and a joint region with the placing member may be reinforced by synthetic leather.

A capsule endoscope medical care system according to another aspect of the present invention includes a capsule endoscope which includes an imaging unit, an illumination unit for illuminating a region to be imaged, and a transmitting unit for transmitting image data obtained by the imaging unit to the outside, a subject being able to swallow the capsule endoscope; a detecting device which is placed in a subject body surface, the detecting device having an antenna structure in which the image data transmitted from the transmitting unit is received as a predetermined electric displacement amount; a portable medical apparatus which is electrically connected to the detecting device through a cable to record the image data received by the detecting device; and the portable medical apparatus storage holder according to the invention which retains the portable medical apparatus on a subject body.

### EFFECT OF THE INVENTION

According to the present invention, the portable medical apparatus storage holder includes the constraint member. The constraint member is continuously connected to the storage container or the placing member, and the constraint member constrains the paths of the cables drawn from the opening of the storage container in which the portable medical apparatus is stored. Therefore, in the vicinity of the storage container, the constraint member constrains the paths of the cables drawn from the opening of the storage container, which results in the following effects: The unstable behavior of the cable is eliminated to prevent the cable jam or looseness, and the steep bending of the cable is eliminated at the base portion to protect the cable.

### BRIEF DESCRIPTION OF DRAWINGS

FIG. 1 is a view showing an entire configuration example of an encapsulated endoscope medical care system including a receiver holder according to a first embodiment of the invention;
FIG. 2 is a perspective view showing an antenna unit and a receiver;
FIG. 3 is a perspective view showing components of the receiver holder of the first embodiment;
FIG. 4 is a perspective view showing an example of the receiver holder in a placed state;
FIG. 5 is a front view showing a state in which the receiver holder is spread out;
FIG. 6 is a rear view showing a state in which the receiver holder is spread out with a flap opened;
FIG. 7 is a rear view showing an example of the receiver holder in the placed state;
FIG. 8 is a perspective view showing a configuration of a part of the receiver holder;
FIG. 9A is an explanatory view showing an example in which a suspender is placed;
FIG. 9B is an explanatory view showing another example in which the suspender is placed;
FIG. 9C is an explanatory view showing still another example in which the suspender is placed;
FIGS. 10A to 10F are views sequentially showing a procedure of placing the receiver holder;
FIG. 11 is a perspective view showing a configuration example of a backside portion of a pouch;
FIG. 12 is a longitudinally sectional side view of a pouch portion;
FIG. 13A is a sectional view taken on line A-A of FIG. 12;
FIG. 13B is a sectional view taken on line B-B of FIG. 12;
FIG. 14 is a perspective view showing a receiver holder according to a second embodiment of the invention;
FIG. 15 is a rear view showing a configuration example of a receiver holder of a first modification;
FIG. 16 is a front view showing a configuration example of the receiver holder of the first modification;
FIG. 17 is a rear view showing a configuration example when a receiver is stored in the receiver holder of the first modification;
FIGS. 18A to 18F are views sequentially showing a procedure of placing the receiver holder;
FIG. 19 is a perspective view showing a configuration example of a receiver holder of a second modification;
FIG. 20 is a perspective view showing a configuration example when the receiver is stored in the receiver holder of the second modification;
FIG. 21 is a rear view showing a configuration example of a receiver holder according to a third embodiment of the invention;
FIG. 22 is a perspective view showing a configuration example when the receiver is stored in the receiver holder of the third embodiment;
FIG. 23 is a front view showing a state in which the receiver holder is placed on a subject body;
FIG. 24 is a side view showing a state in which the receiver holder is placed on the subject body;
FIG. 25 is a perspective view showing a configuration example of a receiver holder according to a fourth embodiment of the invention;
FIG. 26 is a perspective view showing a state in which a cable is pulled in the receiver holder;
FIG. 27 is a longitudinally sectional side view of the receiver holder;
FIG. 28 is a perspective view showing a configuration example in which each portion is opened in a receiver holder according to a fifth embodiment of the invention;
FIG. 29 is a perspective view showing an example in which the cable is drawn in the receiver holder;
FIG. 30 is a perspective view showing a state in which the flap is closed in the receiver holder;
FIGS. 31A to 31F are views sequentially showing a procedure of placing the receiver holder in accordance with a first procedure;
FIGS. 32A to 32F are views sequentially showing the procedure of placing the receiver holder in accordance with a second procedure;
FIG. 33 is a perspective view showing an example in which the cable is drawn in a sixth embodiment of the invention;
FIG. 34 is a partial rear view showing a configuration example of a receiver holder of a third modification;
FIG. 35 is a perspective view showing a configuration example of a receiver holder according to a seventh embodiment of the invention;
FIG. 36 is a perspective view showing a state in which the flap is closed in the receiver holder of the seventh embodiment; and
FIGS. 37A to 37F are views sequentially showing a procedure of placing the receiver holder.

### EXPLANATIONS OF LETTERS OR NUMERALS

- 1: Capsule endoscope medical care system
- 4: Detecting device
- 5: Cable
- 6: Receiver (portable medical apparatus)
- 7 holder): Receiver holder (portable medical apparatus
- 13: Bundling member
- 31: Abdomen belt (abdominal region placing member)
- 41: Pouch (storage container)
- 44: Opening
- 45: Friction member
- 46: Flap (cap member)
- 47: Stitched portion (hinge pair)
- 48: Joint structure (constraint member)
- 49: Friction member
- 56: Groove structure
- 57: Cushioning member (constraint member)
- 61: Suspender (suspension placing member)
- 71 holder): Receiver holder (portable medical apparatus
- 72: Pouch (storage container)
- 73: Abdomen belt (abdominal region placing member)
- 74: Shoulder belt (suspension placing member)
- 75: Opening
- 76: Flap (cap member)
- 77: Stitched portion (hinge pair)
- 80: Synthetic leather
- 91 holder): Receiver holder (portable medical apparatus
- 92: Pouch (storage container)
- 93: Belt (placing member)
- 94: Round string (placing member)
- 95: Opening
- 96: Flap (cap member)
- 97: Stitched portion (hinge pair)
- 100: Synthetic leather
- 103,: 104 Tongue piece (strap member)
- 112: Suspending member (pulling member)
- 121 holder): Receiver holder (portable medical apparatus
- 122: Pouch (storage container)
- 125: Opening
- 127: Flap (cap member)
- 128: Hinge pair
- 132: Auxiliary belt (strap member)
- 135: Cushioning member (constraint member)
- 136: Flap (cap member)
- 137: Hinge pair
- 141 holder): Receiver holder (portable medical apparatus
- 142: Pouch (storage container)
- 143 member): Abdomen flat string (abdominal region placing
- 144: Shoulder flat string (suspension placing member)
- 145: Opening
- 146: Flap (cap member)
- 147: Hinge pair
- 150: Strap member (constraint member)

### BEST MODE(S) FOR CARRYING OUT THE INVENTION

Exemplary embodiments of the invention will be described in detail below with reference to the accompanying drawings.

### First Embodiment

A portable medical apparatus storage holder according to a first embodiment is a receiver holder for retaining a receiver as a portable medical apparatus in a capsule endoscope medical care system.

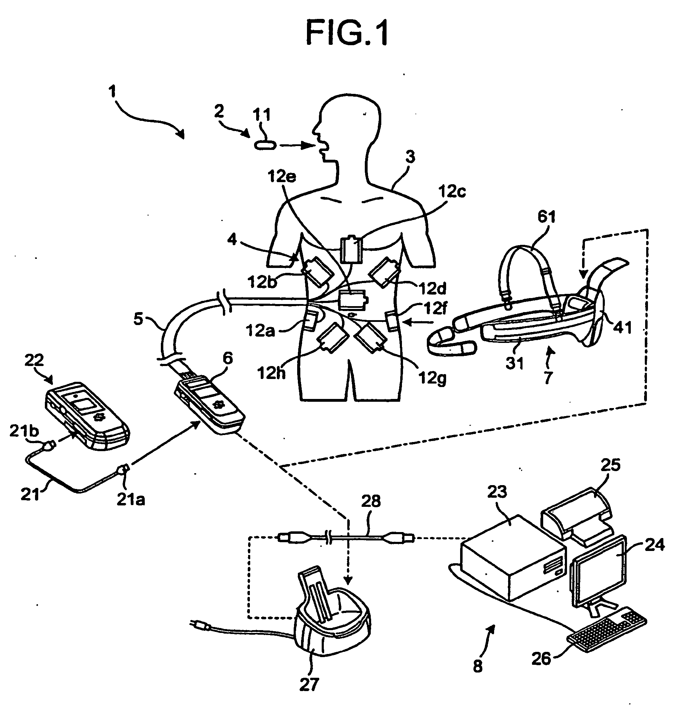

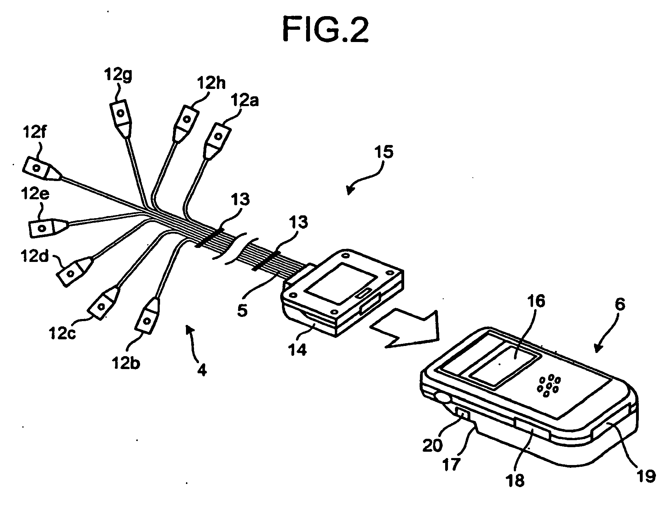

FIG. 1 is a view showing an entire configuration example of a capsule endoscope medical care system including a receiver holder according to a first embodiment, and FIG. 2 is a perspective view showing an antenna unit and a receiver. A capsule endoscope medical care system 1 mainly includes a capsule endoscope 2, a detecting device 4, a receiver 6, a receiver holder 7, and an external unit 8. The detecting device 4 has a loop antenna structure which adheres directly to a predetermined region of a body surface of a subject 3 by bonding or the like. The receiver 6 is the portable medical apparatus which is electrically connected to the detecting device 4 with a cable 5 to record detection result. The receiver holder 7 is the portable medical apparatus storage holder which is placed on the body of the subject 3, e.g., a middle abdomen portion to retain the receiver 6 on the body of the subject 3. The external unit 8 is provided outside the subject 3.

The subject 3 can swallow a capsule 11 of the capsule endoscope 2. An imaging device, an illumination device, a signal processing device, a transmission device, and a power supply which are not shown are incorporated in the capsule 11. When the subject 3 swallows the capsule endoscope 2, the capsule endoscope 2 is introduced into the body cavity. While the capsule endoscope 2 is moved through a conduit in the body cavity, an imaging region illuminated with an illumination device such as LED is taken by an imaging device such as CCD and CMOS to obtain images in the body cavity, image data is converted into predetermined signals by a processing device, and the signals are transmitted to the detecting device 4 by a transmission device.

The detecting device 4 is a receiving antenna which detects the image data signal, wirelessly transmitted from the transmission device in the capsule endoscope 2, as a predetermined electric displacement amount. The detecting device 4 includes plural loop antennas, e.g., eight loop antennas 12a to 12h. The loop antennas 12a to 12h are arranged in predetermined regions of the subject 3 such as right and left flanks, a neighbor of an epigastric fossa, right and left seventh frames, and right and left lower abdomens.

The eight cables 5 extended from the loop antennas 12a to 12h are formed by, e.g., coaxial wire having good shielding properties. Lengths of the cables 5 are previously determined according to each arrangement position of the corresponding loop antennas 12a to 12h on the body surface. As shown in FIG. 2, the eight cables 5 are bundled by plural bundling members 13 so as to be aligned in the same plane from the mid point, and the cables 5 are finally drawn into a rectangular, flat antenna jack unit 14 to be electrically connected to the receiver 6. An antenna unit 15 includes the cables 5, loop antennas 12a to 12h, and the antenna jack unit 14.

The receiver 6 is formed in a slightly flat rectangular shape. As shown in FIG. 2, the receiver 6 includes a liquid crystal display unit 16, an antenna unit bay 17, a viewer cable connector 18, and a cradle port connector 19. A circuit member including a CompactFlash® memory for recording the image data transmitted through the cables 5 is mounted on a board in the receiver 6, and a battery pack having a capacity enough to support the medical care as long as eight to ten hours is installed in a battery chamber in the receiver 6. Therefore, the receiver 6 is formed while having a relatively heavy weight. The antenna unit bay 17 has a connector structure which the antenna jack unit 14 is freely connected to and disconnected from, and the antenna unit bay 17 is configured such that the internal circuit of the receiver 6 and the detecting device 4 are electrically connected to each other while the antenna jack unit 14 is inserted. The numeral 20 designates an eject button for detaching the antenna jack unit 14.

The viewer cable connector 18 is provided on a lower end side in one side face of the receiver 6. A connector 21a of a viewer cable 21 is connected to the viewer cable connector 18 while a connector 21b is connected to a viewer 22, which allows the image recorded in the receiver 6 to be confirmed as needed with the viewer 22 during the medical care.

The receiver holder 7 enables the receiver 6 to be carried by retaining the receiver 6 on the body of the subject 3, e.g., on a left waist region while the loop antennas 12a to 12h are electrically connected to the cables 5. The receiver holder 7 will be described in detail later.

The external unit 8 is mainly formed by a workstation 23 installed in a hospital. The external unit 8 includes a display device 24, a print device 25, and a keyboard 26. The external unit 8 also includes a cradle 27 and a cradle cable 28. The cradle 27 is used such that the workstation 23 collectively captures the image data recorded in the CompactFlash® memory of the receiver 6. The cradle cable 28 is formed by a USB cable or the like. When the receiver 6 is inserted into the cradle 27 to establish the connection state through the cradle port connector 19, the workstation 23 captures all the pieces of image data recorded in the CompactFlash® memory of the receiver 6.

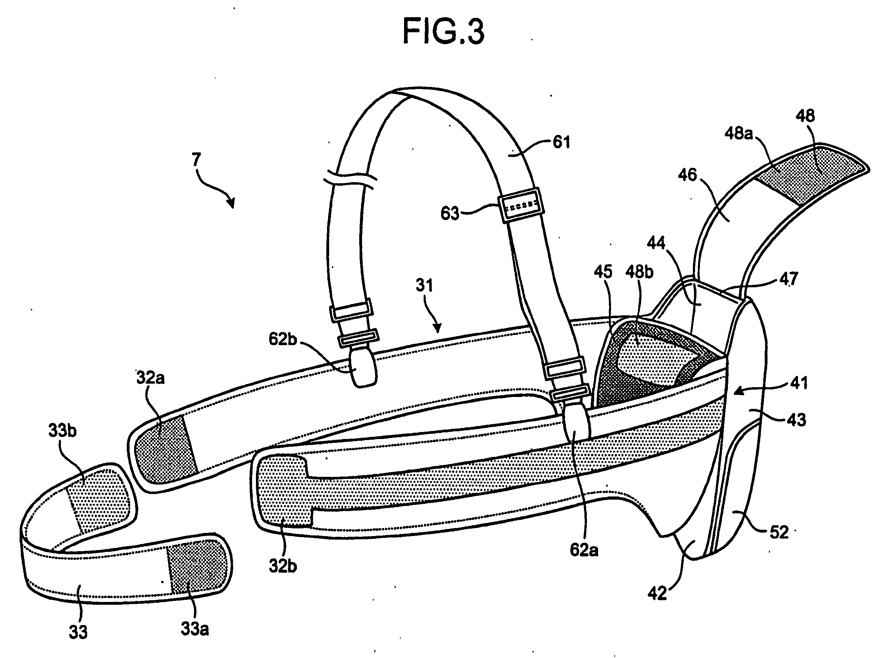

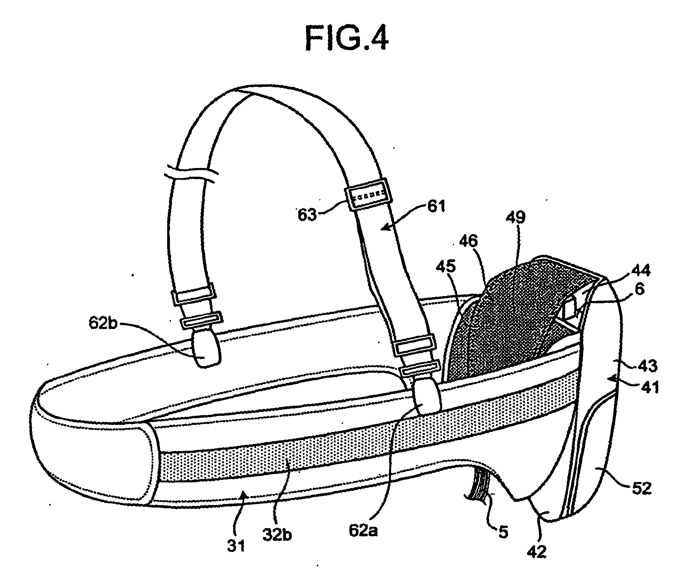

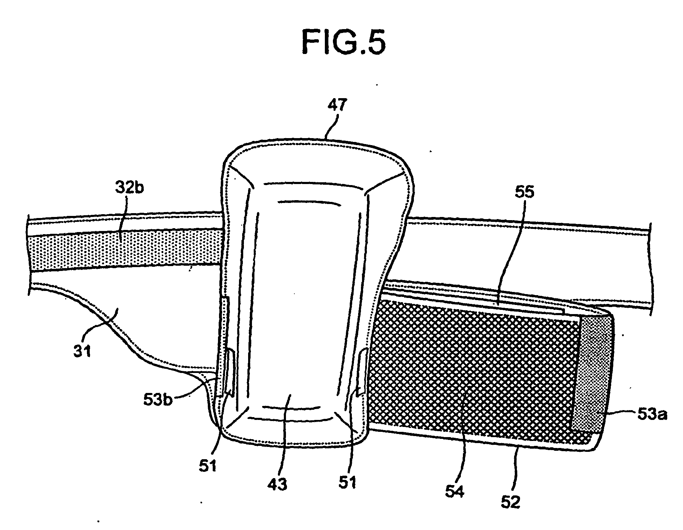

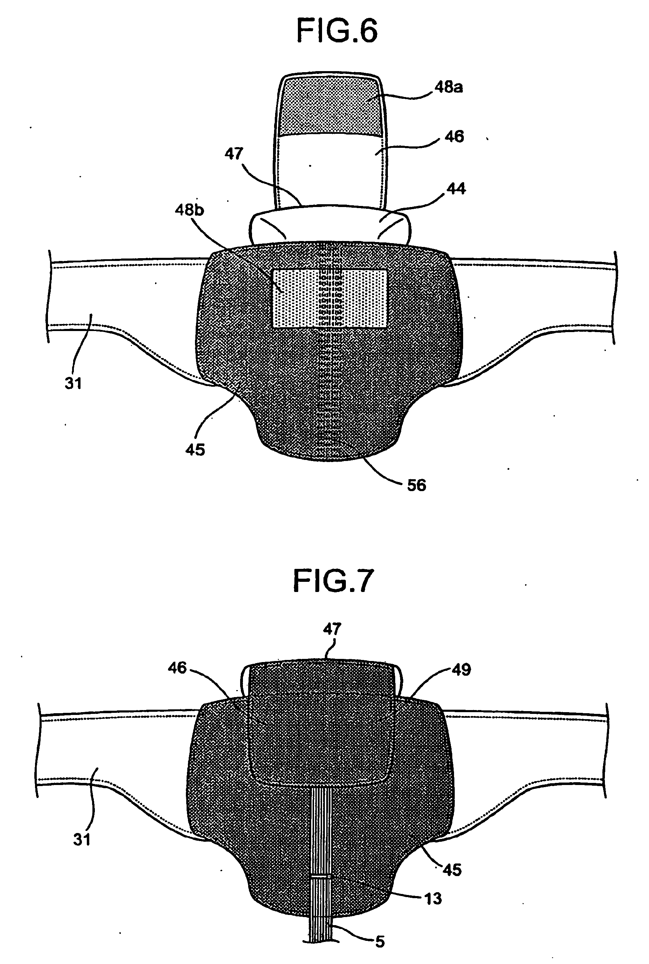

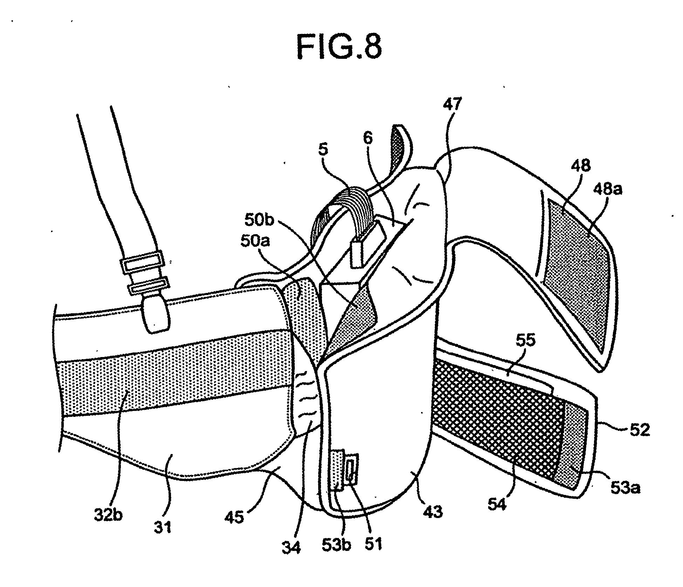

Then, the receiver holder 7 will be described. FIG. 3 is a perspective view showing components of the receiver holder of the first embodiment, FIG. 4 is a perspective view showing an example of the receiver holder in a placed state wherein the subject is not shown, FIG. 5 is a front view showing a state in which the receiver holder is spread out, FIG. 6 is a rear view showing a state in which the receiver holder is spread out with a flap opened, FIG. 7 is a rear view showing an example of the receiver holder in the placed state, and FIG. 8 is a perspective view showing a configuration of a part of the receiver holder. The receiver holder 7 of the first embodiment mainly includes an abdomen belt 31, a pouch 41, and a suspender 61.

The abdomen belt 31 realizes the abdominal region placing member which detachably places the pouch 41 on the body of the subject 3, e.g., the middle abdomen portion. In the first embodiment, the abdomen belt 31 is formed by a cloth belt mainly made of nylon fabrics, and a rim portion of the abdomen belt 31 is stitched with a rim tape made of nylon. The abdomen belt 31 has a basic length, e.g., 90 cm corresponding to a standard abdomen circumference of an adult male. The abdomen belt 31 has a detachable surface fastener structure including a male tape portion 32a and a female tape portion 32b. The male tape portion 32a is stitched on the inside of one end of the abdomen belt 31, and the female tape portion 32b is stitched on the outside of the other end. The abdomen belt 31 is placed around the abdomen portion of the subject 3, the abdomen belt 31 is closed while both the ends overlap each other, and thereby the closed-loop belt is formed. The female tape portion 32b is extended to the neighbor of the belt central portion while a width is narrowed, and the male tape portion 32a is connected to the female tape portion 32b at a desired position. Therefore, even on a child or a female whose abdomen circumference is small, the abdomen belt 31 can be placed because the length is adjustable according to the abdomen circumference. Furthermore, an extension belt 33 whose length is approximately 40 to 50 cm is also prepared for the subject 3 whose abdomen circumference exceeds the standard abdomen circumference. The extension belt 33 has a female tape portion 33b and a male tape portion 33a. The female tape portion 33b is connected to the male tape portion 32a, and the male tape portion 33a is connected to the female tape portion 32b.

The abdomen belt 31 includes a rubber band 34 (see FIG. 8) in the central portion connected to the pouch 41. The rubber band 34 has the length corresponding to the width of the pouch 41. The abdomen belt 31 is formed wider, e.g., the 6-cm width compared with the usual belt such that the stable placement state can be secured with no uncomfortable feeling even if the subject 3 performs bending and stretching movement when the abdomen belt 31 is placed on the subject 3. The portion near the center which is jointed to the rubber band 34 of the abdomen belt 31 is formed in the continuously wide shape such that the subject 3 feels that the pouch 41 is more securely placed at placement region.

The pouch 41 realizes the storage container in which the receiver 6 is detachably stored. The pouch 41 is formed by a long and small cloth bag mainly made of nylon fabrics, and the bag has the shape and size in which the receiver 6 is just stored. Particularly, the pouch 41 is formed by stitching right and left rim portions and lower-end rim portions of a backing cloth portion 42 and a surface cloth portion 43 with a nylon edge tape. The backing cloth portion 42 has a flat shape, and the surface cloth portion 43 has a stereoscopic shape which forms a storage portion. An opening 44 is provided on the upper portion side of the pouch 41. The receiver 6 is inserted and detached through the opening 44, and the cable 5 is drawn from the opening 44. While the length of the backing cloth portion 42 coincides with the length of the receiver 6, the upper portion side of the surface cloth portion 43 is further extended. Therefore, the opening 44 of the first embodiment is positioned while facing the side of the subject 3 when the pouch 41 is placed on the middle abdomen portion of the subject 3.

The pouch 41 is coupled to the abdomen belt 31 by stitching the backing cloth portion 42 and the rubber band 34 in a longitudinal direction. The pouch 41 includes a friction member 45 which constitutes a backside portion of the pouch 41. The friction member 45 is located inside the backing cloth portion 42 and the rubber band 34, and the friction member 45 comes into direct contact with the subject 3 when the pouch 41 is placed. The friction member 45 is filler cloth having a cushioning property (elasticity), and the friction member 45 having the width larger than the width of the pouch 41 while having the same length as the backing cloth portion 42. Therefore, the friction member 45 is formed such that a contact area with the subject 3 is increased in the region where the pouch 41 is placed. The friction member 45 is formed by stitching the rim of the friction member 45 with the nylon edge tape. The cylindrical shape is formed by stitching the upper edge portion and lower edge portion of the friction member 45 and the upper edge portion and lower edge portion of the backing cloth portion 42 respectively, the rubber band 34 of the abdomen belt 31 is passed through the inside of the cylindrical shape, and the central portion of the friction member 45 is stitched in the longitudinal direction along with the backing cloth portion 42 and the rubber band 34. Therefore, the friction member 45 is integral with the pouch 41 and the abdomen belt 31.

Specifically, the fabric of the filler cloth constituting the friction member 45 is made of mesh-shape polyester woven fabric. For example, THELMA (product name of TOKYO SINCO LEATHER CO., Ltd.) is used as the friction member 45. THELMA is formed thick, and THELMA has a rich cushioning property in which the material deforms softly. THELMA is curved while abutting on the cloths of the subject 3, so that THELMA can increase contact area to secure frictional force having a level at which the friction member 45 is hardly slips and slides.

A flap 46 which is the cap member for opening and closing the opening 44 is provided on the upper end side of the pouch 41. One end of the flap 46 is stitched to a side on the top portion side of the surface cloth portion 43 forming the opening 44, and the stitched portion 47 serves as the hinge pair. The flap 46 is configured to be opened and closed while the opening 44 is covered with the backside of the flap 46. The flap 46 has a size in which an end side parallel to the hinge pair (stitched portion 47), i.e., a front edge overlaps the pouch fabric below the opening 44 on an outer surface on the backside of the pouch 41 when the opening 44 is closed. In the first embodiment, the flap 46 has the size in which the flap 46 overlaps an upper half of the friction member 45 constituting the backside portion of the pouch 41. In the overlapping range, a male tape portion 48a having a proper size and shape is stitched on the inner surface side of the flap 46, and a female tape portion 48b is stitched at the corresponding position on the outer surface side of the friction member 45. Therefore, a joint structure 48 which includes the surface fastener for attachment and detachment is configured.

The flap 46 is formed by stitching the rim of the flap 46 with the nylon edge tape. A friction member 49 having the cushioning property (elasticity) is provided over the whole surface of the flap 46 which comes into contact with the body of the subject 3 when the flap 46 is closed, i.e., over the whole outer surface. The friction member 49 is made of the same material as the friction member 45. For example, THELMA (product name of TOKYO SINCO LEATHER CO., Ltd.) is used as the friction member 49.

In the pouch 41, the side edge portions of the backing cloth portion 42 and surface cloth portion 43 are not stitched up to the opening 44, but stitched to the midpoint from the lower end side, and thereby the upper end portion continued to the opening 44 is configured to be freely opened and expanded as shown in FIG. 8. Therefore, in storing the receiver 6 in the pouch 41, the size of the opening 44 is expanded to facilitate the storage of the receiver 6. The expanded portion is formed by the surface fastener structure including a male tape portion 50a and a female tape portion 50b.

In the right and left sides of the pouch 41, connection ports 51 for connecting the connector 21a of the viewer cable 21 are formed at positions corresponding to the viewer cable connector 18 of the receiver 6 stored. A cover member 52 which covers the connection ports 51 is provided in the surface of the pouch 41. The cover member 52 is also mainly made of the nylon fabric, and cover member 52 is formed by stitching the rim of the cover member 52 with the nylon edge tape. Both ends of the cover member 52 may be formed so as to be detachably attached to the pouch 41. In the first embodiment, the right side of the cover member 52 is integral with the pouch 41 by the stitching, and the left side is freely opened and closed by the surface fastener structure including a tape portion 53a and a female tape portion 53b. Therefore, loss of the cover member 52 is prevented. In the cover member 52, a pocket 54 having a mesh structure is provided on the inner surface side, and a small object such as a memo 55 can be stored in the pocket 54. Therefore, the small object such as the memo 55 in which emergency contact numbers are listed can be easily carried outside the home.

The reason why a priority is given to the left connection port 51 while the connection ports 51 are provided on the right and left sides of the pouch 41 will be described. Frequently a doctor tracks the state of the subject 3 while seeing the viewer 22 until the capsule endoscope 2 reaches a duodenum through a stomach since the subject 3 swallows the capsule endoscope 2. In this case, the subject 3 takes a body position in which the subject 3 lies on the right waist side so that the capsule endoscope 2 reaches easily the duodenum located on the right side of the body. Therefore, the pouch 41 is basically placed on the left waist so as not to stand in the way. However, sometimes the pouch 41 cannot be placed on the left waist depending on the subject 3. In the first embodiment, assuming that the pouch 41 might be placed on the right waist, the connection ports 51 are provided on both the sides in order to enable the viewer to be connected to the receiver 6 from both right and left sides of the pouch 41. In the case where the pouch 41 is placed on the right waist, the orientation of the receiver 6 may be turned to store the receiver 6 in the pouch 41. Because the pouch 41 is basically placed on the left waist, the left side is freely opened and closed and the priority is given to the connection port 51 on the left side in the attachment structure of the cover member 52.

The suspender 61 will be described below. The suspender 61 realizes the placing member as the suspension placing member which is suspended on the shoulder of the subject 3 to place the pouch 41 on the middle abdomen portion of the subject 3. The suspender 61 is not limited to an 1-shape one-belt type, an 1-shape two-belt type, a Y-shape belt type, an H-shape belt type, and the like. In the first embodiment, the suspender 61 is formed in the I-shape one-belt type. Because the receiver holder 7 is placed on body of the subject 3 in the ordinary life, there is a demand that the suspender maintains a low posture depending on cloths, and a female feels uncomfortable when the two suspenders are passed through a chest portion. This is because the suspender 61 is formed in the I-shape one-belt type. The suspender 61 is a flat string to which a stretching property is given by rubber. Clip 62a and 62b which are engageable with the abdomen belt 31 at an arbitrary position are provided at both ends of the suspender 61, and an adjuster 63 for adjusting the length is arranged at mid point.

Therefore, the suspender 61 can freely be placed in position with respect to the abdomen belt 31, the suspender 61 can be placed in length according to the body shape of the subject 3 by adjusting the adjuster 63, and various attachment states can be adopted according to preference of the subject 3. The suspender 61 is not always required. However, as described later, an operation for storing the receiver 6 in the pouch 41 is performed while the abdomen belt 31 is loosened, and preferably the suspender 61 is included in order to retain the pouch 41 on the body even if the abdomen belt 31 is loosened.

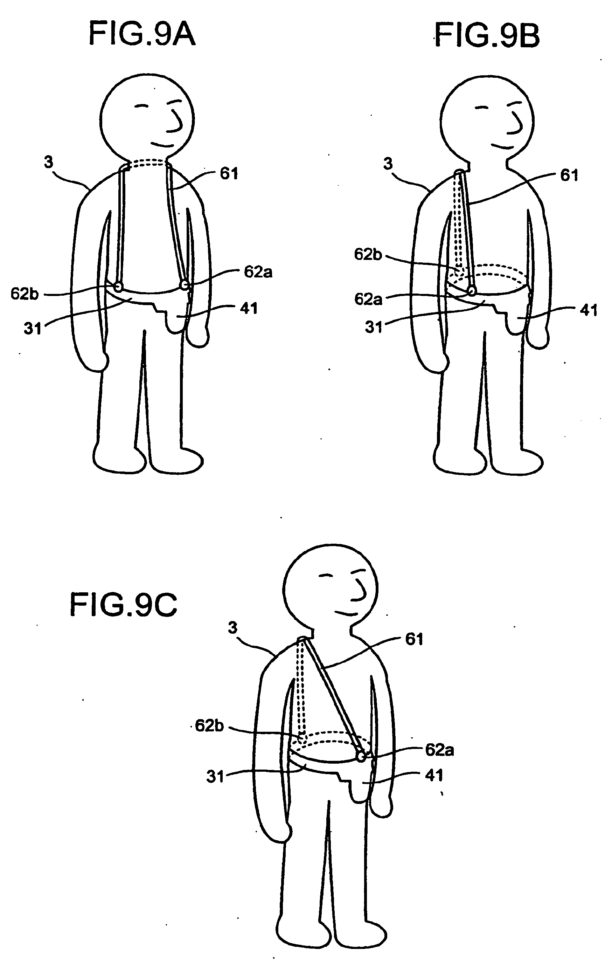

FIGS. 9A to 9C are explanatory views showing several example in which a suspender is placed. FIG. 9A shows an example in which both ends of the suspender 61 are fastened at the front side of the abdomen belt 31 by clips 62a and 62b while the suspender 61 is folded at shoulders (around a neck). FIG. 9B shows an example in which the suspender 61 is placed in parallel with an axis line of the body only by the right shoulder. FIG. 9C shows an example in which the suspender 61 is obliquely placed on the body from the right shoulder to a left flank.

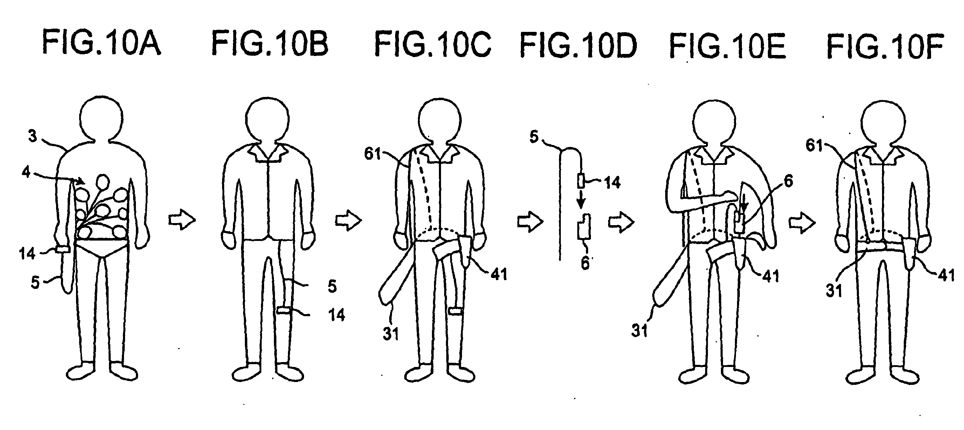

The procedure of placing the receiver holder 7 of the first embodiment on the subject 3 will be described below. FIGS. 10A to 10F are views sequentially showing a procedure of placing the receiver holder 7. As shown in FIG. 10A, the loop antennas 12a to 12h adhere to proper points on the body surface of the subject 3. This treatment is performed by the doctor. As shown in FIG. 10B, the subject 3 wears the cloths. At this point, the antenna jack unit 14 is in a standby state while hanging from the end of the cloths. Then, the clips 62a and 62b at both the ends of the suspender 61 are placed at desired positions of the abdomen belt 31, and the abdomen belt 31 is placed around the abdomen while the suspender 61 is suspended on the shoulder as shown in FIG. 10C. However, at this point, the surface fasteners at both the ends are not closed yet.

As shown in FIG. 10D, the receiver 6 is picked up, and the antenna jack unit 14 is connected to the antenna unit bay 17 of the receiver 6. At this point, the power switch of the receiver 6 is turned on to confirm whether or not the power lamp is normally lit in green. Then, as shown in FIG. 10E, the flap 46 is opened while the pouch 41 is slightly tilted forward, and the receiver 6 is stored into the pouch 41 through the opening 44.

The cover member 52 is opened once to connect the connector 21a of the viewer cable 21 to the viewer cable connector 18. The capsule endoscope 2 is started up, and the capsule endoscope 2 is brought close to the loop antenna on the chest to confirm that a display lamp of the receiver 6 is changed from the turn-off state to the normal green blinking state. At the same time, it is confirmed that the image is displayed on the viewer 22. After the confirmation, the subject 3 really swallows the capsule 11, and the confirmation that the capsule 11 reaches the stomach is made with the viewer 22. After the confirmation, the viewer cable 21 is disconnected from the receiver 6 to close the cover member 52.

After the confirmation, as shown in FIG. 10F, while the pouch 41 is slightly tilted forward, the flap 46 is closed such that the cable 5 drawn onto the backside (body side) from the opening 44 is clamped by the joint structure 48 including the male tape portion 48a and the female tape portion 48b. The surface fastener portions at both the ends of the abdomen belt 31 are closed while jointed to each other at the desired length position, which secures the correct attachment state with respect to the middle abdomen portion of the subject 3. The length of the suspender 61 is also appropriately adjusted.

Then, the subject 3 leads the ordinary life while the pouch 41 in which the detecting device 4 and the receiver 6 are stored is placed on the body for eight to ten hours until the medical care is finished. During the medical care, the radio wave transmitted from the capsule 11 swallowed by the subject 3 is caught by the loop antennas 12a to 12h adhering to the eight points in the body surface of the subject 3, the caught data is transmitted to the receiver 6 through the antenna cable 5, and the data is recorded in the CompactFlash® memory of the receiver 6. During the medical care, if needed, the cover member 52 is opened to connect the connector 21a of the viewer cable 21 to the viewer cable connector 18, and thereby the current image data can be confirmed with the viewer 22. When the subject 3 brings the receiver 6 from which the antenna unit 15 is disconnected to the hospital at the time the measurement is finished with the capsule endoscope 2, in the hospital, the receiver 6 is inserted into the cradle 27, and the workstation 23 connected to the cradle 27 captures all the pieces of measurement data recorded in the receiver 6 through the USB cable 28. Then, the doctor observes the measurement result as a moving picture on the display device 24 of the workstation 23.

As described above, according to the receiver holder 7 of the first embodiment, the opening 44 of the pouch 41 is positioned so as to face the side of the subject 3 while the pouch 41 is placed on the subject 3, so that the cable 5 drawn from the opening 44 is orientated toward the body side of the subject 3. Therefore, the cable 5 can be drawn so as to be hidden behind the backside of the pouch 41 or the abdomen belt 31, the cable 5 can be hidden when viewed from the direction in front of the outer surface of the pouch 41, and the cable 5 can be protected against the external force.

According to the receiver holder 7 of the first embodiment, the pouch 41 includes the flap 46 which freely opens and closes the opening 44, which allows the easy protection of the cable 5 drawn from the opening 44. Particularly, the flap 46 is configured to be freely open and close the opening 44 toward the backside of the pouch 41 by the hinge pair (stitched portion 47) located on the upper side. Therefore, the cable 5 can be drawn from the opening 44 in the backside portion of the pouch, the cable 5 can be protected by the flap 46, and the cable 5 extending outside the flap 46 can be suitable stored in the cloths. In this case, the cable 5 drawn onto the backside (body side) from the opening 44 is clamped by the joint structure 48 in which the surface fastener including the male tape portion 48a and the female tape portion 48b is used, the looseness of the drawn cable 5 can be prevented.

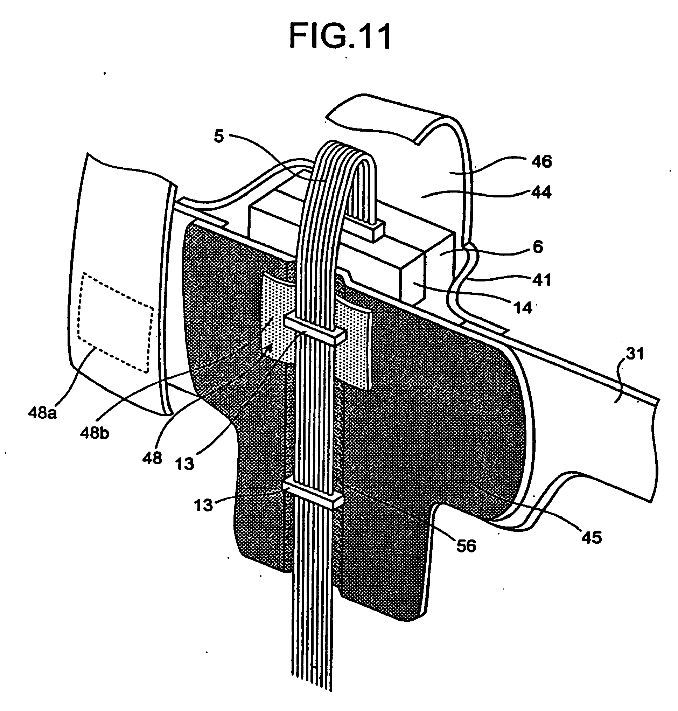

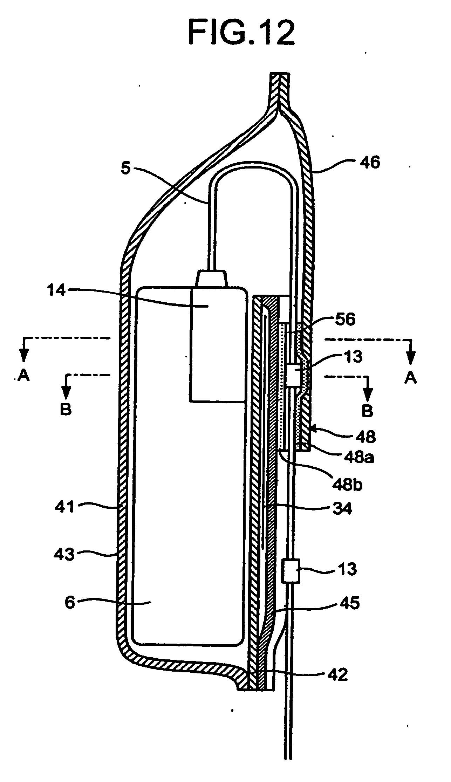

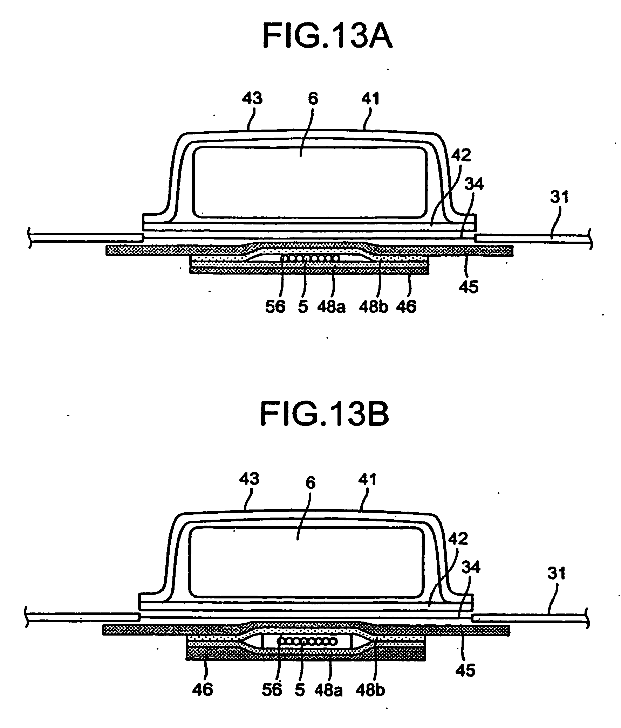

A structure for clamping the cable 5 at the joint structure 48 as the constraint member will be descried in detail with reference to FIGS. 6 and 11 to 13B. FIG. 11 is a perspective view showing a configuration example of a backside portion of the pouch 41, FIG. 12 is a longitudinally sectional side view of the pouch 41, FIG. 13A is a sectional view taken on line A-A of FIG. 12, and FIG. 13B is a sectional view taken on line B-B of FIG. 12. In the first embodiment, the friction member 45 is integral with the pouch 41 and the abdomen belt 31 by stitching the central portion of the friction member 45 in the longitudinal direction along with the backing cloth portion 42 and the rubber band 34. The female tape portion 48b is also stitched together, and the stitched portion is formed not by longitudinal one line but by several lines, which allows the vertical formation of the concave groove structure 56 having slightly wide recess shape in the central portion of the friction member 45. In the formation of the groove structure 56, the thick friction member 45 having the rich cushioning property (elasticity) is tightened by stitching the friction member 45 and the backing cloth portion 42, and a dent is made in the friction member 45. The groove width of the groove structure 56 is set to the size in which the bundling member 13 for bundling the eight cables 5 can be fitted.

In the case where the receiver 6 is stored in the pouch 41 while the cable 5 is drawn onto the backside from the opening 44, as shown in FIG. 11, the bundling member 13 closest to the receiver 6 is fitted at the position close to the upper end of the groove structure 56 in the female tape portion 48b, and the eight cables 5 are fitted in the groove structure 56. Then, the flap 46 is closed to joint the male tape portion 48a to the female tape portion 48b. Therefore, the bundling member 13 and the cable 5 are clamped by the joint structure 48 in which the surface fastener is used, so that the looseness is prevented in the horizontal direction. At the same time, the rectangular reed-shaped bundling member 13 is clamped by the joint structure 48 in which the surface fastener is used, so that the looseness can also be prevented in the lengthwise direction of the cable 5. As a result, in the path of the base portion of the cable 5 drawn from the receiver 6, the proper setting of the retaining position of the bundling member 13 by the joint structure 48 can constrain the cables 5 without steeply bending the cable 5 from the base portion such that the cable 5 is drawn upward from the base portion once and then bent as shown in FIGS. 11 and 12. Therefore, the degradation can be prevented at the base portion in the cable 5 having the characteristic that the core wire is easily disconnected by the metal fatigue due to the repeated bending. Particularly, because the bundling member 13 and the cable 5 are clamped by the joint structure 48 in which the surface fastener is used while fitted in the groove structure 56, as shown in FIGS. 13A and 13B, the jointing property between the male tape portion 48a and the female tape portion 48b becomes better around the bundling member 13 and the cable 5, and the slip preventing effect is increased.

According to the receiver holder 7 of the first embodiment, in the configuration around the pouch 41, the flap 46 is located on the backside which is the body side of the subject 3, and the abdomen belt 31 and the like are also provided on the backside with respect to the pouch 41. Therefore, the inner peripheral side is formed in a flat shape over all, and the placement can be performed with a good sense of the fit. Particularly, in the vicinity of the pouch 41, the abdomen belt 31 is continuously formed in the wide shape, so that the sense of the fit is further obtained. In the vicinity of the pouch 41, the rubber band 34 is arranged in the abdomen belt 31, and the friction member 45 (the friction member 49 for the flap 46) which has the length equal to the height of the pouch 41 and the width larger than that of the pouch 41 exists in the backside portion of the pouch 41 which is in contact with the cloths. Therefore, when the abdomen belt 31 is placed around the abdomen portion, the pouch 41 does not slide nor slip, and the surface contact state in which the load is not concentrated but evenly distributed is obtained with respect to the waist portion. Accordingly, the pouch 41 in which the receiver 6 is stored is adapted to the body in the common motion of the subject 3, and the sense of the fit can be maintained.

According to the receiver holder 7 of the first embodiment, in the receiver holder 7, the flap 46 is tightened by the abdomen belt 31 when the receiver holder 7 is placed on the body, so that the flap 46 can be opened when the abdomen belt 31 is loosened. Therefore, during the medical care in which the receiver 6 is carried, the receiver 31b does not fall from the pouch 41 or the receiver 31b is not exposed by carelessly opening the flap 46, and the malfunction of the receiver 31 can be prevented. The mistaken operation performed by the subject 3 can also be prevented.

### Second Embodiment

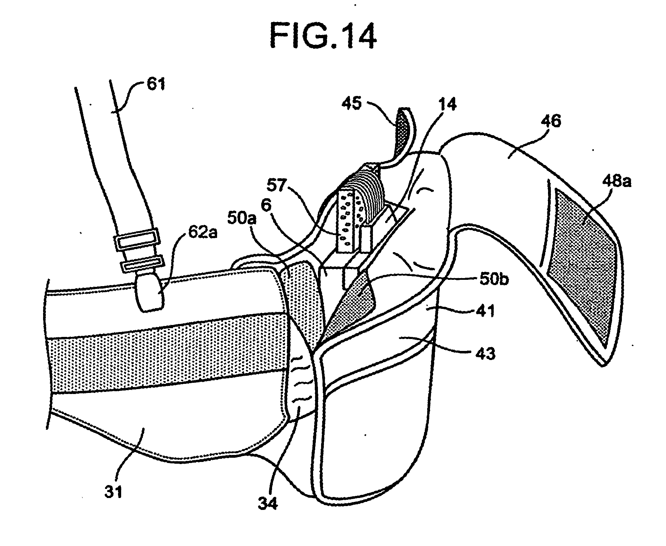

FIG. 14 is a perspective view showing a receiver holder according to a second embodiment. In the second embodiment, the upper end side of the backing cloth portion 42 constituting the pouch 41 is extended upward to increase the height, and a cushioning member 57 as the constraint member for constraining the path of the cable 5 drawn from the opening 44 of the pouch 41 is provided by fixing the cushioning member 57 to the upper portion on the inner surface side of the extended backing cloth portion 42. The cushioning member 57 is located on the upper-end backside of the receiver 6 stored in the pouch 41. That is, the cushioning member 57 is arranged at the position surrounded by the bent cable 5 in the region where the base portion of the cable 5 drawn onto the backside of the pouch 41 from the upper end of the receiver 6 through the opening 44 is bent, and the cushioning member 57 regulates the path of the cable 5 such that a bending radius is increased in the base portion of the cable 5. For example, the cushioning member 57 is made of a urethane foam material, and the cushioning member 57 is formed in a simple rectangular shape.

In the second embodiment, in the case where the cable 5 drawn to the backside from the receiver 6 stored in the pouch 41 through the opening 44 of the pouch 41, when the cable 5 is drawn so as to be bent on the cushioning member 57, the cable 5 can be constrained by the cushioning member 57 such that the cable 5 is not steeply bent toward the backside portion from the end portion, but the cable 5 is bent after the cable 5 is drawn upward from the end portion. Therefore, the degradation caused by the steep bending can be prevented at the base portion in the cable 5 having the characteristic that the core wire is easily disconnected by the metal fatigue due to the repeated bending.

### First Modification

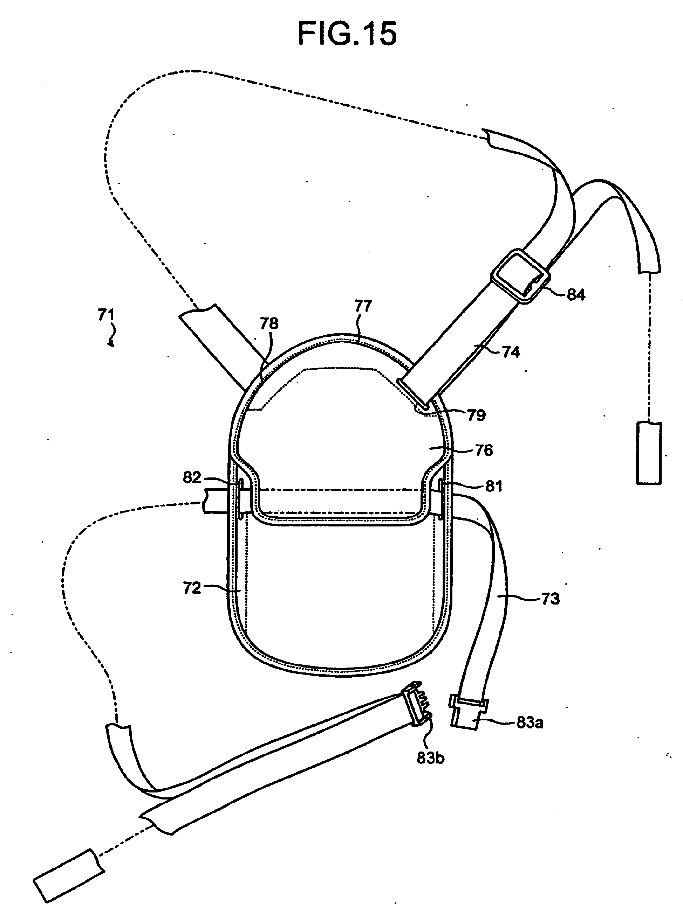

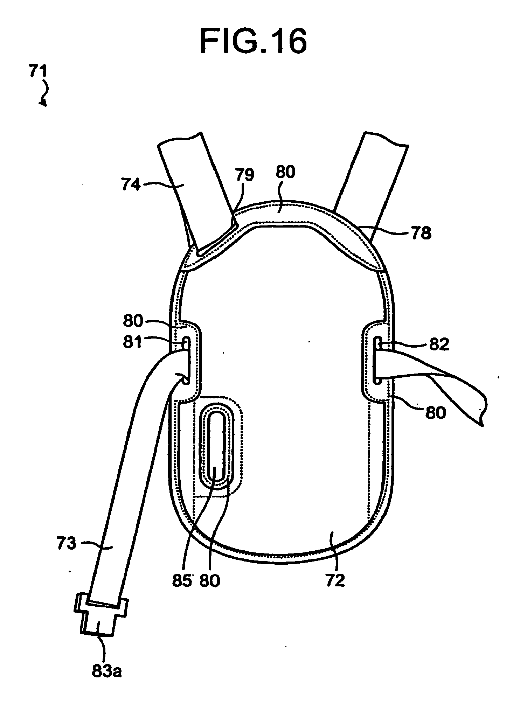

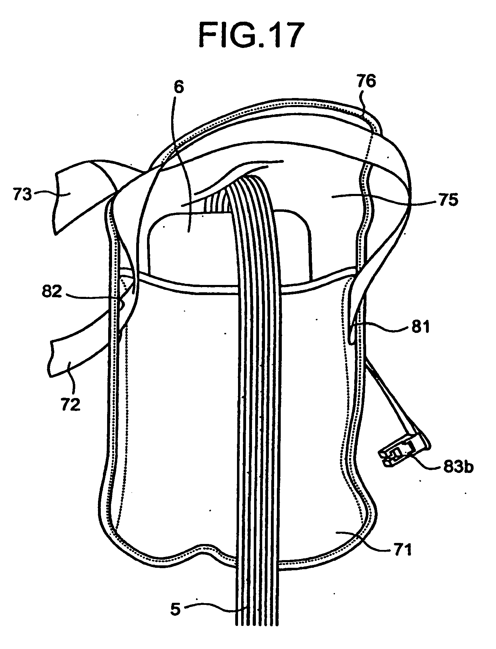

FIG. 15 is a rear view showing a configuration example of a receiver holder of a first modification, FIG. 16 is a front view showing a configuration example of the receiver holder of the first modification, and FIG. 17 is a rear view showing a configuration example when the receiver is stored in the receiver holder of the first modification. Unlike the reuse type first embodiment and second embodiment, a receiver holder 71 of a first modification is formed as a disposable type. This is because, in the medical site, there is a demand not to reuse but dispose the receiver holder which is already used by another patient (subject) from the standpoint of hospital infection prevention.

The receiver holder 71 of the first modification mainly includes a pouch 72, an abdomen belt 73, and a shoulder belt 74. The pouch 72, the abdomen belt 73, and the shoulder belt 74 are mainly formed by nonwoven cloths which is suitable to the disposable use. As shown in FIG. 17, an opening 75 is provided on the upper portion side of the pouch 72 as the storage container, and the opening 75 is positioned so as to face the side of the subject 3 when the pouch 72 is placed on the body of the subject 3. The opening 75 is covered with a flap 76 as the cap member while freely opened and closed. The flap 76 is integral with the opening 75 of the pouch 72 by stitching the upper side of the flap 76 to the side on the top portion side of the opening 75 of the pouch 72, and a stitched portion 77 of the flap 76 is formed as the hinge pair while freely opened and closed toward the pouch backside.

The pouch 72 has a shoulder belt joint portion 78 at an upper right corner portion when viewed from the front face, and one end of the shoulder belt 74 is jointed to the shoulder belt joint portion 78 by the stitching. A shoulder belt through hole 79 through which the other end of the shoulder belt 74 is passed is made in the upper left corner portion of the pouch 72 when viewed from the front face. The shoulder belt joint portion 78 and shoulder belt through hole 79 which are of the joint region are reinforced by a synthetic leather 80. Abdomen belt through holes 81 and 82 through which an abdomen belt 73 is passed are made in the central portion on the both sides of the pouch 72, and the abdomen belt through holes 81 and 82 which are of the connection region are reinforced by the synthetic leather 80. The rim of the pouch 72 is formed by the stitching with the nylon edge tape.

The abdomen belt 73 which is of the abdominal region placing member made of nonwoven cloth is formed in the long and thin belt shape. The abdomen belt 73 is drawn through the abdomen belt through holes 81 and 82 on the backside of the pouch 72, and a female side buckle 83a is attached to one end of the abdomen belt 73. The abdomen belt 73 is arranged so as to be passed through the inner surface side of the front end which is of the end side parallel to the hinge pair of the flap 76, and the abdomen belt 73 is continuously connected to the inner surface of the front edge portion of the flap 76 by the stitching so as to be integral with the flap 76. The other end side of the abdomen belt 73 is folded such that the abdomen belt 73 has the total length enough to strap the abdomen circumference irrespective of the physical size. A male side buckle 83b which is detachably attached to a female side buckle 83a is provided in the folded portion of the abdomen belt 73. The male side buckle 83b is also used as the adjuster. The abdomen belt 73 is passed through the adjuster near the folded portion where the two abdomen belts 73 overlap each other, and thereby the length is freely adjusted by the adjuster. The nonwoven cloth which is of the material of the abdomen belt 73 is a material which is easily cut with the scissors as a cutting tool, and the excessive nonwoven cloth generated by the length adjustment can be removed by the cutting.

The shoulder belt 74 which is of the suspension placing member is made of the nonwoven cloth, and the shoulder belt 74 is formed in the long and thin belt shape so as to have the length enough to be suspended on the shoulder independently of the physical size. One end of the shoulder belt 74 is jointed to the shoulder belt joint portion 78 of the pouch 72 by the stitching. On the other end side, the two shoulder belts 74 overlapping each other are passed through an adjuster 84 after the shoulder belt 74 is passed through the shoulder belt through hole 79, and thereby the length is freely adjusted by the adjuster 84. The nonwoven cloth which is of the material of the shoulder belt 74 is a material which is easily cut with the scissors as the cutting tool, and the excessive nonwoven cloth generated by the length adjustment can be removed by the cutting.

In the case where the image is observed with the viewer 22 during the medical care, the connector 21a of the viewer connecting cable 21 is connected to the viewer cable connector 18 of the receiver 6, and a connection port 85 for connecting the connector 21a is formed in position of the pouch 72. The connection port 85 is also reinforced by the synthetic leather 80.

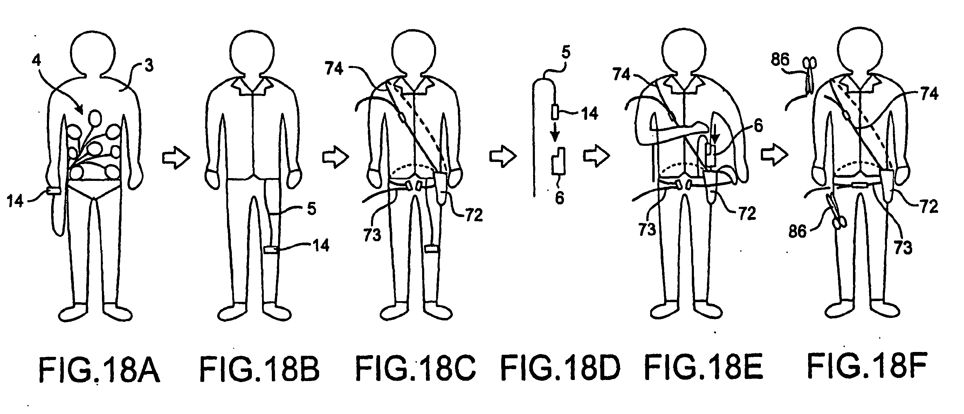

The procedure of placing the receiver holder 71 of the first modification on the subject 3 will be described below. FIGS. 18A to 18F are views sequentially showing the procedure of placing the receiver holder 71. As shown in FIG. 18A, the loop antennas 12a to 12h adhere to proper points on the body surface of the subject 3. This treatment is performed by the doctor. As shown in FIG. 18B, the subject 3 wears the cloths. At this point, the antenna jack unit 14 is in the standby state while hanging from the end of the cloths. As shown in FIG. 18C, the shoulder belt 74 is suspended on the shoulder. However, at this point, the male side buckle 83b and female side buckle 83a of the waist belt 73 are not latched yet.

As shown in FIG. 18D, the receiver 6 is picked up, and the antenna jack unit 14 is connected to the antenna unit bay 17 of the receiver 6. At this point, the power switch of the receiver 6 is turned on to confirm whether or not the power lamp is normally lit in green. Then, as shown in FIG. 18E, when the flap 76 of the pouch 72 is opened, the wide opening 75 surrounded by the abdomen belt 73 associated with the front end of the flap 76 is opened as shown in FIG. 17, the receiver 6 is stored in the pouch 72 through the opening 75. The cable 5 is drawn from the opening 75 so as to be passed through the portion clamped between the front edge of the flap 76 and the backside of the pouch 72.

At this point, the connector 21a of the viewer cable 21 is connected to the viewer cable connector 18.
The capsule endoscope 2 is started up, and the capsule endoscope 2 is brought close to the loop antenna on the chest to confirm that a display lamp of the receiver 6 is changed from the turn-off state to the normal green blinking state. At the same time, it is confirmed that the image is displayed on the viewer 22. After the confirmation, the subject 3 really swallows the capsule 11, and the confirmation that the capsule 11 reaches the stomach is made with the viewer 22. After the confirmation, the viewer cable 21 is disconnected from the receiver 6.

After the confirmation, as shown in FIG. 18F, the male side buckle 83b and the female side buckle 83a are engaged with each other, the abdomen belt 73 is pulled to conform to the abdomen circumference of the subject 3 by the adjuster of the male side buckle 83b, and the excessive portion is cut and removed by the scissors 86. On the side of the shoulder belt 74, the length is appropriately adjusted, and the excessive portion is cut and removed by the scissors 86.

As described above, according to the first modification, the abdomen belt 73 and shoulder belt 74 having the sufficient length irrespective of the physical size are prepared, so that the placement state suitable to the physical size of the subject 3 can be secured by the method of simply cutting the excessive portion which is unique to the disposable type, and the length adjustment mechanism can be simplified. Although the pouch 72 is made of the inexpensive nonwoven cloth, the joint region with the abdomen belt 73 or the shoulder belt 74 is reinforced by the synthetic leather 80, so that the stable placement state can be maintained.

According to the first modification, the abdomen belt 73 passed through the abdomen belt through holes 82 and 81 is continuously connected by the stitching so as to be integral with the front edge portion of the flap 76 which closes the opening 75. Therefore, when the side of the flap 76 is placed on the body to tighten the abdomen belt 73, the pouch 72 can abut strongly on the body side while the cable 5 drawn between the flap 76 and the pouch 72 is strongly pressed against the side of the pouch 72 (side of receiver 6), so that the stable placement state can be obtained.

### Second Modification

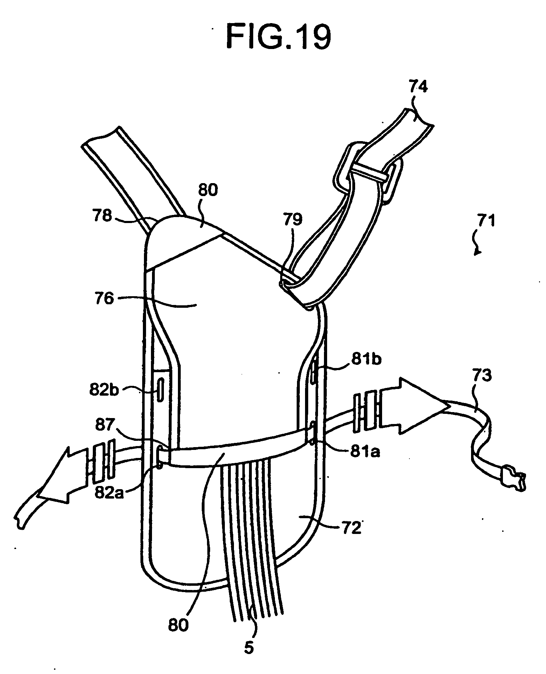

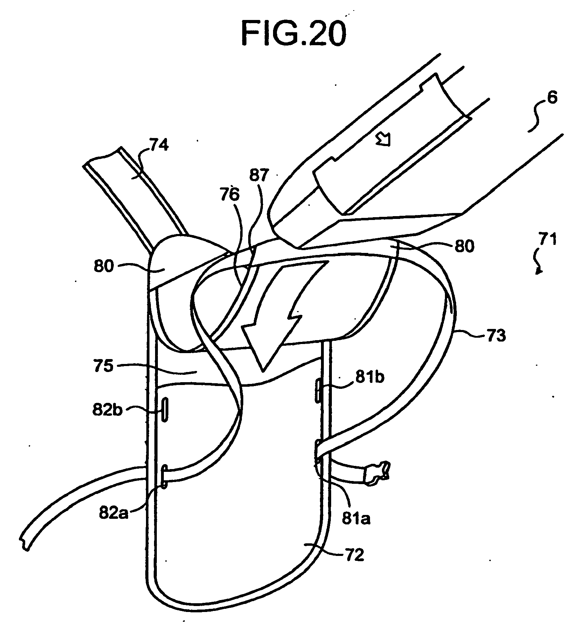

FIG. 19 is a perspective view showing a configuration example of a receiver holder of a second modification, and FIG. 20 is a perspective view showing a configuration example when the receiver is stored in the receiver holder of the second modification. The receiver holder 71 of the second modification is similar to the receiver holder of the first modification, and the same component is designated by the same numeral. In the pouch 72 of the receiver holder 71 of the second modification, the abdomen belt through holes 82 and 81 through which the abdomen belt 73 is passed are formed at two different positions in the vertical direction as shown by the numerals 82a, 82b, 81a, and 81b. A cylindrical sleeve portion 87 which is folded and stitched is formed in the front edge portion of the flap 76 such that the abdomen belt 73 is freely passed through the sleeve portion 87, and the sleeve portion 87 is reinforced by assigning the synthetic leather 80 to the sleeve portion 87. Therefore, in the third modification, the abdomen belt 73 is continuously connected to the front edge portion of the flap 76 by inserting the abdomen belt 73 into the sleeve portion 87.

The pouch 72 can be placed on the middle abdomen portion at the position corresponding to the physical size and preference of the subject 3 by appropriately selecting the case where the abdomen belt 73 is inserted into the abdomen belt through holes 82a and 81a and the case where the abdomen belt 73 is inserted into the abdomen belt through holes 82b and 81b. Even if the abdomen belt 73 is inserted into the different points, because the abdomen belt 73 is continuously connected to the front edge portion of the flap 76, the stable placement state can be obtained like the first modification when the abdomen belt 73 is tightened.

### Third Embodiment

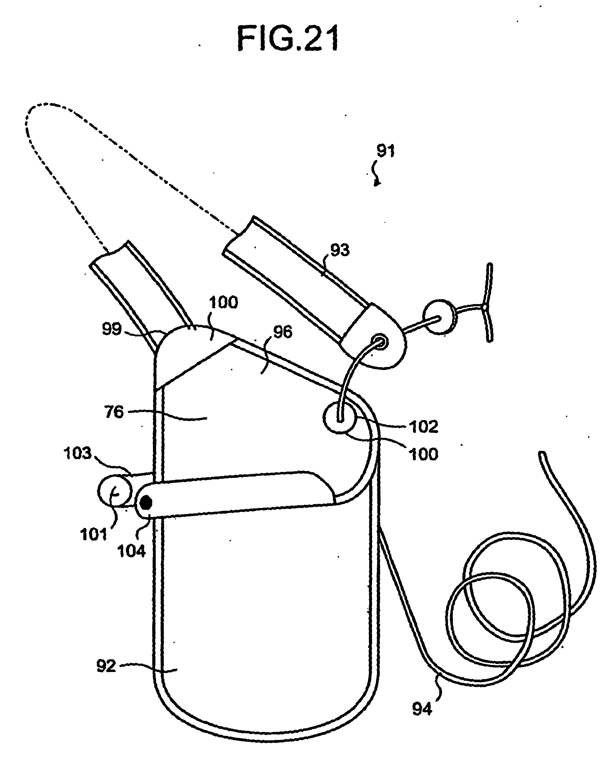

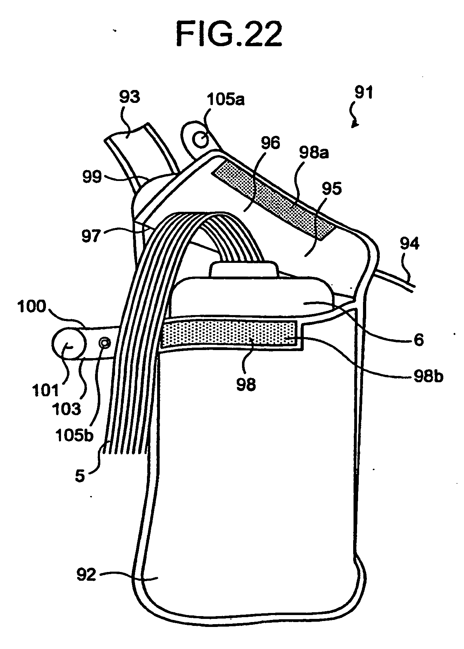

FIG. 21 is a rear view showing a configuration example of a receiver holder according to a third embodiment of the invention, and FIG. 22 is a rear view showing a configuration example when the receiver is stored in the receiver holder of the third embodiment. A receiver holder 91 of the third embodiment includes a disposable nonwoven-cloth pouch 92, a disposable nonwoven-cloth belt 93, and a disposable round string 94.

The pouch 92 which is of the storage container is formed by the stitching with the nylon edge tape. As shown in FIG. 22, an opening 95 is provided on the upper portion side of the pouch 92, and the opening 95 is located so as to face the side of the subject 3 when the pouch 92 is placed on the body of the subject 3. The pouch 92 is covered with a flap 96 as the cap member while freely opened and closed. The upper side of the flap 96 is integral with the side on the top portion side of the opening 95 of the pouch 92 by the stitching, and a stitched portion 97 serves as the hinge pair. The flap 96 is configured to be opened and closed toward the pouch backside. The front end side of the flap 96 has a joint structure 98 formed by the surface fastener having a male tape 98a and a female tape 98b, and the front end side of the flap 96 is detachably attached to the pouch 92.

The placing member is a combination of the belt 93 and the round string 94. The belt 93 is formed into a length having an extent in which the length exceeds a distance between the waist portion of the subject 3 and the shoulder portion on the opposite side. One end of the belt 93 is integrally jointed to a belt joint portion 99 on the upper left end of the pouch 92 by the stitching when viewed from the backside. A belt joint portion 99 is reinforced by synthetic leather 100. The other end of the belt 93 is reinforced by the synthetic leather 100, and one end of the round string 94 is latched in a non-slip manner. The round string 94 has the total length enough to strap the abdomen circumference independently of the physical size of the subject 3.

String through holes 101 and 102 are made at two points in the pouch 92. The string through hole 101 is formed by a fitting at the front edge of a tongue piece 103 made of the synthetic leather 100, and the tongue piece 103 is horizontally extended at the position corresponding to the front edge of the flap 96 while traversing the front face of the pouch 92. The string through hole 102 is located on the side opposite to the string through hole 101 with respect to the pouch 92, and the string through hole 102 is made at such a position that the belt joint portion 99 and the string through holes 101 and 102 form a substantial triangle with the belt point portion 99 as the vertex. The string through hole 102 is reinforced by the synthetic leather 100. A tongue piece 104 made of the synthetic leather 100 is integrally provided by the stitching on the front-edge surface side of the flap 96, and the tongue piece 104 is horizontally extended at the position corresponding to the tongue piece 103 while being shorter than the tongue piece 103. A female hook 105a and a male hook 105b are provided at the front edge of the tongue piece 104 and the corresponding position of the tongue piece 103 respectively, and the tongue pieces 104 and 103 are detachably attached to each other. The tongue pieces 103 and 104 function as the strap member.

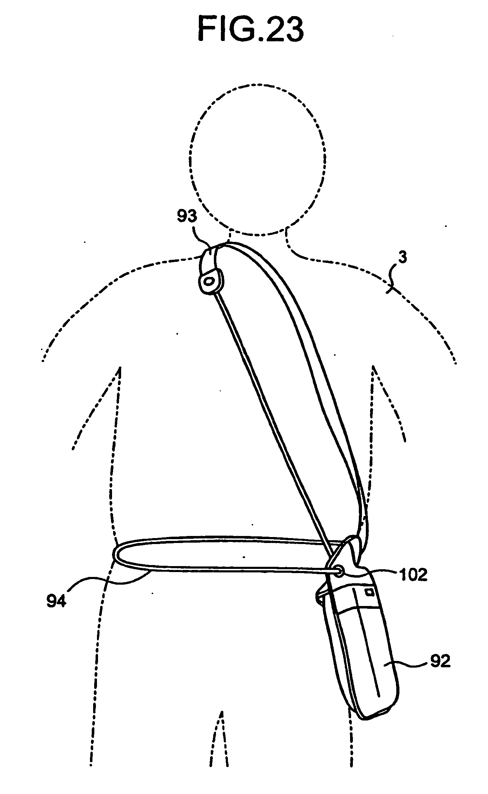

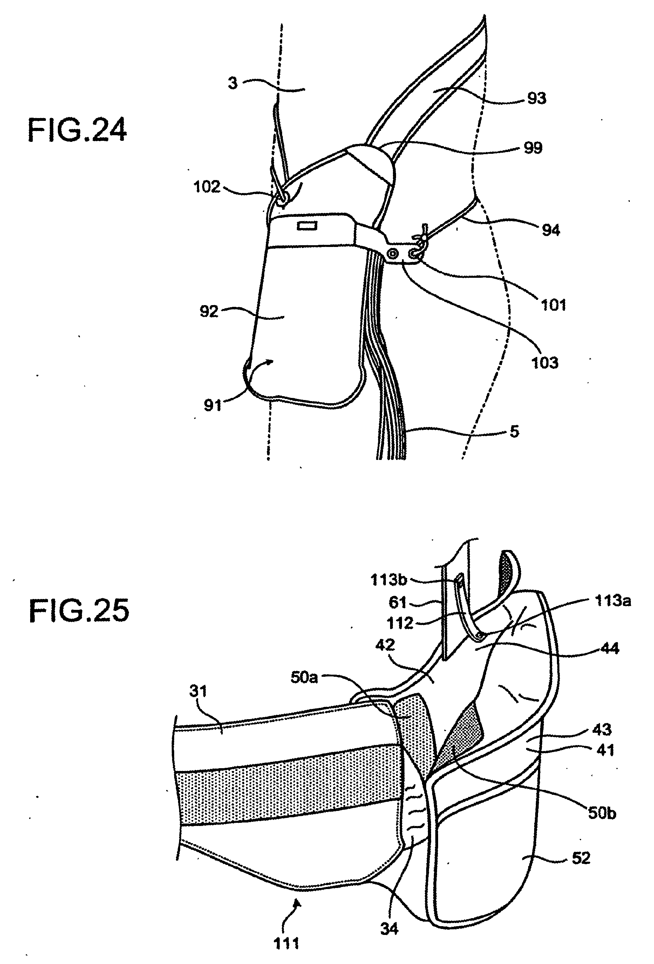

The procedure of placing the receiver holder 91 of the third embodiment will be described below. FIG. 23 is a front view showing a state in which the receiver holder 91 is placed on the subject body 3, and FIG. 24 is a side view showing a state in which the receiver holder 91 is placed on the subject body 3. The belt 93 passes over the backside of the subject 3, and extends over the right shoulder of the subject 3 to the front side so as to held the round strip 94 which passes through the string through hole 102, and the round string 94 is tentatively wound around the string through hole 102. In this state, as described above, the flap 96 is opened to store the receiver 6 in the pouch 92 through the opening 95, and the flap 96 is closed. Then, the tentatively wound round string 94 is released, the round string 94 is passed through the backside from the abdomen and strapped around the abdomen portion, the round string 94 is passed through the string through hole 101, the round string 94 is tied around the string through hole 101, and the excessive portion is cut and removed by the scissors or the like as shown in FIG. 24.

The cable 5 drawn from the opening 95 is basically passed through the joint structure 98. When the cable is passed through the side-face side of the pouch 92 as shown in FIG. 22, the drawn cable 5 can be guided to the side of the pouch 92 with the tongue pieces 103 and 104 by engaging the male hook 105b and the female hook 105a, so that the jam of the drawn cable 5 can be prevented.

In the third embodiment, the placing member is formed by the combination of the belt 93 and the round string 94. Alternatively, the placing member may be formed only by the round string. However, when the placing member includes the belt 93, which is placed at least on the shoulder portion, the weight of the pouch 92 in which the receiver 6 is stored can be received in a planar manner by the shoulder. Because most of the placing member is formed by the round string 94, contact pressure is decreased, the placement can be performed with no sense of the protrusion, and the placing member is inconspicuous. Therefore, the placing member is suitable for the female.

In the third embodiment, the pouch 92 is supported by the three points of the belt joint portion 99 and the string through holes 101 and 102, a gravity center position of the receiver 6 stored in the pouch 92 is set so as to be located above a gravity center position of the pouch 92 of itself, and the belt joint portion 99 is pulled upward by the belt 93. Therefore, the looseness of the receiver 6 (pouch 92) which is placed and carried on the body of the subject 3 can be prevented to maintain the placement state in which the receiver 6 is in close contact with the body.

### Fourth Embodiment

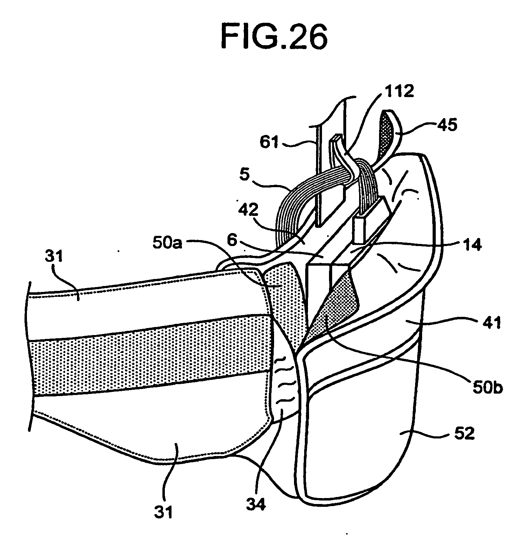

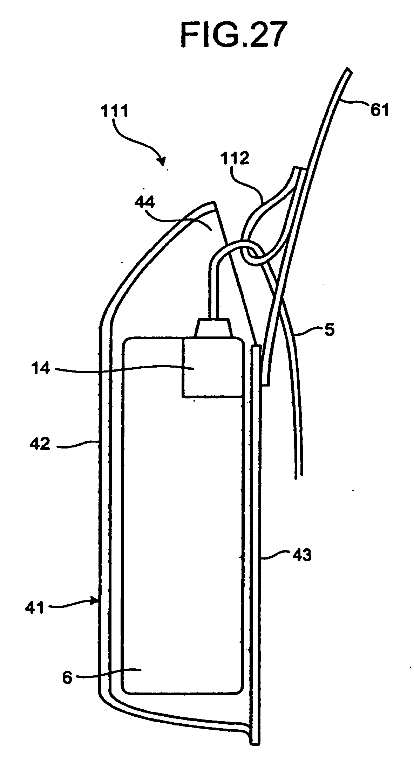

FIG. 25 is a perspective view showing a configuration example of a receiver holder according to a fourth embodiment of the invention, FIG. 26 is a perspective view showing a pulled-in state of a cable in the receiver holder, and FIG. 27 is a longitudinally sectional side view of the receiver holder. The same component as the first embodiment is designated by the same numeral.

In the structure of the pouch 41 of a receiver holder 111 according to the fourth embodiment, the pouch 41 has the opening 44 positioned so as to face the side of the subject 3 when the pouch 41 is placed on the body of the subject 3, although the pouch 41 does not have the flap 46. One end of the suspender 61 which is of the suspension placing member is connected integrally and continuously by the stitching to the upper end of the backing cloth portion 42 located on the backside of the pouch 41. A suspension belt 112 which is of the pulling member is attached to the suspender 61 by the stitching, and the suspension belt 112 is located above the upper end of the receiver 6 stored in the pouch 41. Both ends of the suspension belt 112 have the surface fastener structures in which a male tape portion 113a and a female tape portion 113b are detachably engaged with each other, and the suspension belt 112 can be formed in a loop shape having the size in which the eight cables 5 are bundled by engaging the male tape portion 113a and female tape portion 113b.

In the above configuration, after the receiver 6 is stored in the pouch 41, the cable 5 is drawn from the opening 44 to the backside of the pouch. The neighbor of the base portion of the drawn cables 5 are passed through the suspension belt 112 provided in the suspender 61, the male tape portion 113a and the female tape portion 113b are engaged with each other, and the neighbor of the base portion of the drawn cables 5 are retained in the pulled state by the suspension belt 112 having the loop shape. Therefore, the path of the cable 5 drawn from the opening 44 is constrained so as to be passed through the loop-shape suspension belt 112, and the cable 5 runs toward the backside of the abdomen belt 31. Because the cable 5 is pulled by the suspension belt 112 by utilizing the fact that the cable 5 has the strong durability against the tensile load, the cable 5 is not steeply curved from the end portion toward the backside, but the cable 5 can be curved after the cable 5 is once drawn upward from the end portion. Therefore, the degradation can be prevented at the base portion in the cable 5 having the characteristic that the core wire is easily disconnected by the metal fatigue due to the repeated bending.

### Fifth Embodiment

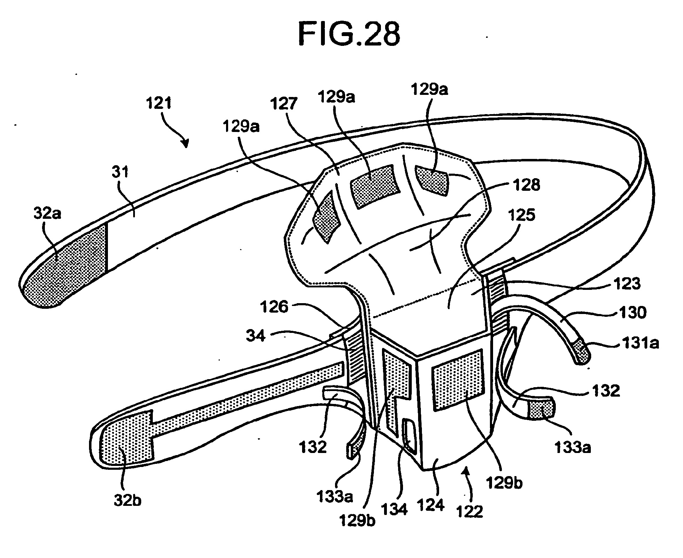

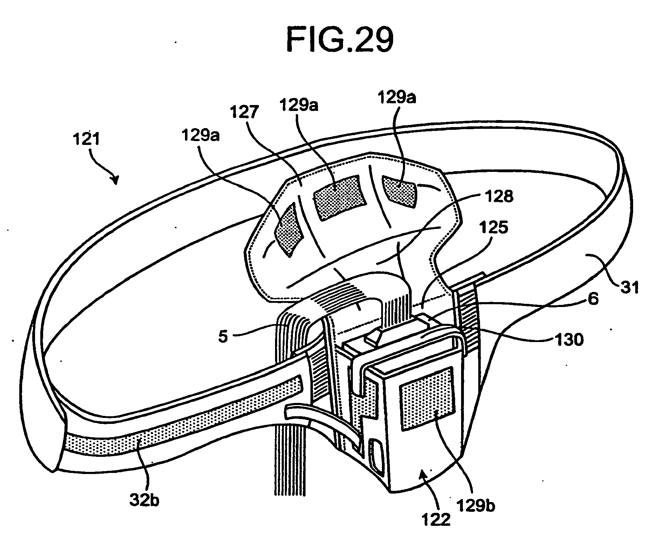

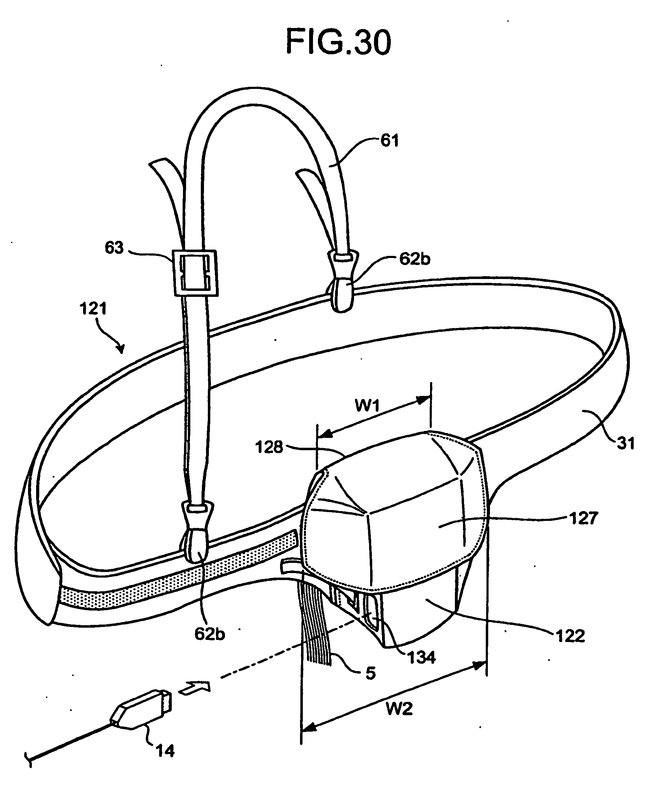

FIG. 28 is a perspective view showing a configuration example of a receiver holder according to a fifth embodiment in which each portion is opened, FIG. 29 is a perspective view showing an example in which the cable is drawn in the receiver holder, and FIG. 30 is a perspective view showing a state in which the flap is closed in the receiver holder. The same component as the first embodiment is designated by the same numeral.

In a receiver holder 121 of the fifth embodiment, a pouch 122 differs from the pouch 41 in the configuration, while the abdomen belt 31 and the suspender 61 are similar to those of the first embodiment. The pouch 122 realizes the storage container in which the receiver 6 is detachably stored. The pouch 122 is formed by a long and small cloth bag mainly made of the nylon fabrics, and the bag has the shape and size in which the receiver 6 is just stored. Particularly, the pouch 122 is formed by stitching the right and left rim portions and the lower-end rim portions of a backing cloth portion 123 and a surface cloth portion 124 with the nylon edge tape. The backing cloth portion 123 has the flat shape, and the surface cloth portion 124 has the stereoscopic shape which forms the storage portion. An opening 125 is provided in the upper end portion of the pouch 122. The receiver 6 is inserted and detached through the opening 125, and the cable 5 is drawn from the opening 125.

The pouch 122 is coupled to the abdomen belt 31 by stitching the backing cloth portion 123 and the rubber band 34 in the longitudinal direction. The pouch 122 includes a friction member 126 which constitutes the backside portion of the pouch 122. The friction member 126 is located inside the backing cloth portion 123 and the rubber band 34, and the friction member 126 comes into direct contact with the subject 3 when the pouch 122 is placed. The friction member 126 is the filler cloth having the cushioning property (elasticity), and the friction member 126 has the width larger than the width of the pouch 122 while having the same length as the pouch 122. Therefore, the friction member 126 is formed such that the contact area for the subject 3 is increased in the region where the pouch 122 is placed. The friction member 126 is formed by stitching the rim of the friction member 126 with the nylon edge tape. The cylindrical shape is formed by stitching the upper edge portion and lower edge portion of the friction member 126 and the upper edge portion and lower edge portion of the backing cloth portion 123 respectively, the rubber band 34 of the abdomen belt 31 is passed through the inside of the cylindrical shape, and the central portion of the friction member 126 is stitched in the longitudinal direction along with the backing cloth portion 123 and the rubber band 34. Therefore, the friction member 126 is integral with the pouch 122 and the abdomen belt 31.

Specifically, the fabric of the filler cloth constituting the friction member 126 is made of mesh-shape polyester woven fabric. For example, THELMA (product name of TOKYO SINCO LEATHER CO., Ltd.) is used as the friction member 126. THELMA is formed thick, and THELMA has a rich cushioning property in which the material deforms softly. THELMA is curved while abutting on the cloths of the subject 3, so that THELMA can increase contact area to secure frictional force having a level at which the friction member 126 is hardly slips and slides.

A flap 127 which is of the cap member for opening and closing the opening 125 is provided on the upper end side of the pouch 122. The flap 127 is continuously and integrally connected with the same width as the pouch 122 to the top portion side of the backing cloth portion 123 constituting the opening 125, and the flap 127 has the structure in which the flap 127 is opened and closed with a fulcrum of a hinge pair 128 while the opening 125 is covered with the front side of the flap 127. That is, the flap 46 of the first embodiment is the backside opening type while the flap 127 of the fifth embodiment is the front-face opening type. The flap 127 has the size and bending shape in which the front edge overlaps the pouch fabric below the opening 125 in the outer surface on the front-face side of the pouch 122 when the opening 125 is closed. In the overlapping range, a female tape portion 129b having the proper size and shape is stitched at several points in the outer surface side of the surface cloth portion 124, e.g., front-face upper portion and upper portions on both sides in the horizontal direction, and a male tape portion 129a is stitched at the corresponding position on the inner surface side of the flap 127. Therefore, a joint structure 129 including the surface fastener for attachment and detachment is configured.

The flap 127 is formed in the size and outline shape so as to hide the cable 5 drawn from the opening 125 to the backside through the right or left side of the hinge pair 128 when the pouch 122 is placed on the body, in front-face projection in which the pouch 122 is viewed from the direction in front of the outer surface on the front-face side of the pouch 122. The flap 127 is formed such that a width W2 of the front-face side overlapping portion where the flap 127 overlaps the surface cloth portion 124 is larger than a width W1 of the hinge pair 128 on the backside, That is, the width W2 on the front-face projection of the flap 127 is larger than the width of the backing cloth portion 123 of the pouch 122.

The pouch 122 includes a pressing band 130 as the pressing member which partially covers the opening 125 by horizontally traversing the opening 125 at the pouch upper-end portion when the flap 127 is opened. One end of the pressing band 130 is fixed to the right side face of the opening of the pouch 127 by the stitching, and a male tape portion 131a of the surface fastener provided in the other end is detachably engaged with the female tape portion 129b.

The receiver holder 121 of the fifth embodiment includes plural auxiliary belts 132 as the strap member. One end of the auxiliary belt 132 is fixed to a proper point of the abdomen belt 31 by the stitching, a male tape portion 133a of the surface fastener provided on the free end of each auxiliary belts 132 is detachably engaged with the female tape portion 129b. The loop structure formed by the auxiliary belt 132 which is closed by jointing the male tape portion 133a to the female tape portion 129b is set at a proper diameter for bundling the eight cables 5. The numeral 134 designates a connection port for connecting the connector 21a of the viewer cable 21.

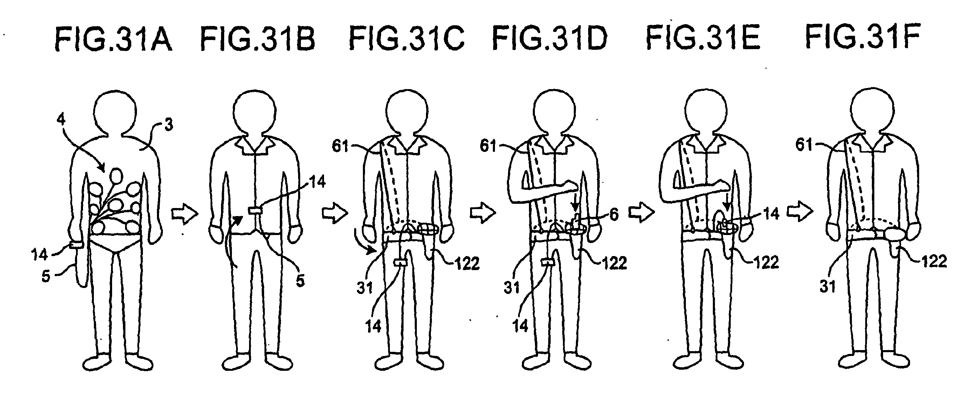

A first procedure of placing the receiver holder 121 of the fifth embodiment on the subject 3 will be described below. FIGS. 31A to 31F sequentially show the procedure of placing the receiver holder 121. The first procedure will be described as an example of the case where the subject 3 places the abdomen belt 31 so as to cover the cable 5 from the outside. As shown in FIG. 31A, the loop antennas 12a to 12h adhere to proper points on the body surface of the subject 3. This treatment is performed by the doctor. As shown in FIG. 31B, the subject 3 wears the cloths. At this point, although the antenna jack unit 14 is in the standby state while hanging from the end of the cloths, the antenna jack unit 14 is lifted up to the position above the abdomen belt 31 prior to the placement of the abdomen belt 31. As shown in FIG. 31C, while the suspender 61 is suspended on the shoulder, the abdomen belt 31 is placed by winding the abdomen belt 31 around the middle abdomen portion from above the cable 5 to close both the ends. After the abdomen belt 31 is placed, the antenna jack unit 14 is hung on the front side of the abdomen belt 31.

As shown in FIG. 31D, the receiver 6 whose antenna bay 17 is opened is stored in the pouch 122. The flap 127 is kept open. Then, as shown in FIG. 31E, the antenna jack unit 14 is attached to the antenna bay 17 of the receiver 6 stored in the pouch 122. The cable 5 is drawn to the side of the subject 3 from the opening 125 through a convenient side of the right and left sides of the hinge pair 128 as shown in FIGS. 29 and 31F, and the flap 127 is closed as shown in FIG. 30. The pressing band 130 is closed before the flap 127is closed.

As described above, according to the receiver holder 121 of the fifth embodiment, the flap 127 for opening and closing the opening 125 at the upper end of the pouch 122 in which the receiver 6 is stored has the shape which hides the cable 5 drawn to the side of the subject 3 from the opening 125 of the pouch 122 in which the receiver 6 is stored. Therefore, even if the cable 5 drawn from the opening 125 to the side of the subject 3 is exposed, as shown in FIG. 30, the cable 5 can be hidden behind the flap 127 by closing the flap 127 when viewed from the direction in front of the outer surface on the front-face side of the pouch 122, so that the cable 5 can be protected against the external force and the appearance quality can be ensured. The cable 5 does not emerge in front of the abdomen belt 31 in the placement state, and the cable 5 is pressed against the body by the abdomen belt 31, so that the looseness is not generated in the cable 5.

The receiver holder 121 of the fifth embodiment is configured to include the flap 127 which opens toward the front side. Therefore, the operation for storing the receiver 6 in the pouch 122 can be performed while the abdomen belt 31 is wound around the abdomen circumference of the subject 3, so that the placement procedure becomes comprehensible. The receiver holder 121 of the fifth embodiment also has the pressing band 130 which traverses the opening 125 of the pouch 122. Therefore, the upper end of the receiver 6 stored in the pouch 122 can be pressed by the pressing band 130, so that the drop-out of the receiver 6 can be avoided even if the front-face open type flap 127 is carelessly opened.

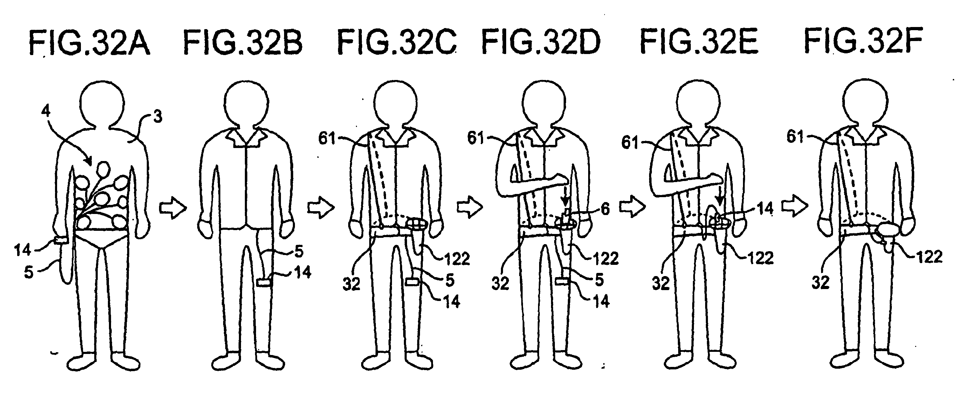

A second procedure of placing the receiver holder 121 of the fifth embodiment on the subject 3 will be described below. FIGS. 32A to 32F sequentially show the procedure of placing the receiver holder 121. The second procedure will be described as an example of the case where the abdomen belt 31 is placed so that the abdomen belt 31 is located inside the cable 5. As shown in FIG. 32A, the loop antennas 12a to 12h adhere to proper points on the body surface of the subject 3. This treatment is performed by the doctor. As shown in FIG. 32B, the subject 3 wears the cloths. At this point, the antenna jack unit 14 is in the standby state while hanging from the end of the cloths. As shown in FIG. 32C, while the suspender 61 is suspended on the shoulder, the abdomen belt 31 is placed by winding the abdomen belt 31 around the middle abdomen portion to close both the ends.

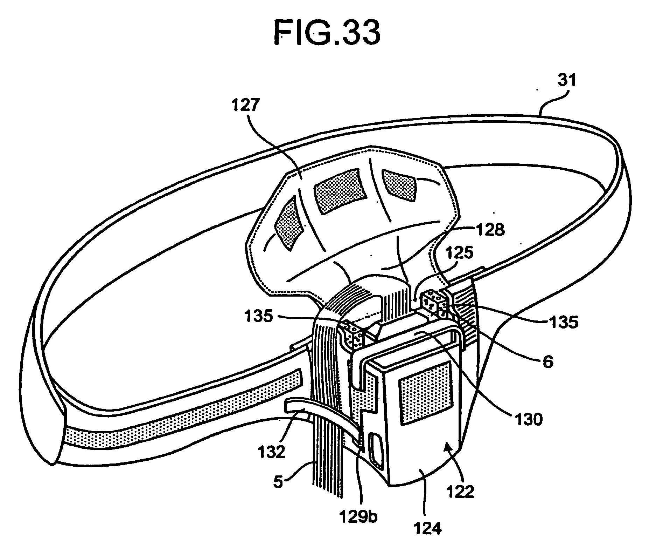

As shown in FIG. 32D, the receiver 6 whose antenna bay 17 is opened is stored in the pouch 122. The flap 127 is kept open. Then, as shown in FIG. 32E, the antenna jack unit 14 is attached to the antenna bay 17 of the receiver 6 stored in the pouch 122 while the cable 5 is passed through the outside (front-face side) of the abdomen belt 31. As shown in FIG. 33, the cable 5 is drawn from the opening 125 of the pouch 122 along the left side (or right side), and the cable 5 is wound and surrounded on the outer surface side of the pouch 122 by closing the auxiliary belt 132 on the side where the cable 5 is drawn. Then, the flap 127 is closed as shown in FIG. 32F. The pressing band 130 is closed before the flap 127 is closed.

The first procedure is recommended as the procedure of placing the receiver holder 121 of the fifth embodiment. However, because it is difficult to give the information on the first procedure to all the various kinds of subjects, sometimes the subject adopts the second procedure in which the cable 5 is placed outside the abdomen belt 31 after the abdomen belt 31 is tightened. Even if the cable 5 is unintentionally placed by the second procedure, the path of the cable 5 which is drawn from the opening 125 and exposed outside the pouch 122 is constrained to the outer surface of the pouch 122. Therefore, the looseness of the exposed cable 5 is prevented, and the unexpected external force is prevented from being applied to the cable 5, so that a lifetime of the cable 5 can be lengthened.

### Sixth Embodiment

A sixth embodiment will be described with reference to FIG. 33. FIG. 33 is a perspective view showing an example in which the cable is drawn in a sixth embodiment of the invention. In the sixth embodiment, cushioning members 135 which are of the constraint member for constraining the path of the cable 5 drawn to the right or left side from the opening 125 of the pouch 122 are provided while fixed to the inner-surface upper portions on both sides in the horizontal direction of the pouch 122 by a bonding agent or the like. The upper end side of the surface cloth portion 124 to which the cushioning members 135 are fixed is extended upward. The cushioning members 135 are located on the right and left positions of the upper end of the receiver 6 stored in the pouch 122. That is, the cushioning members 135 are arranged at the positions surrounded by the bent cable 5 in the region where the base portion of the cable 5 drawn onto the right or left side of the pouch 122 from the upper end of the receiver 6 through the opening 122 by the second procedure is bent, and the cushioning members 135 regulate the path of the cable 5 such that the bending radius is increased in the base portion of the cable 5. For example, the cushioning member 135 is made of the urethane foam material, and the cushioning member 135 is formed in the simple rectangular shape.

In the sixth embodiment, in the case where the cable 5 is drawn onto the right or left side from the receiver 6 stored in the pouch 122 through the opening 125 of the pouch 122, when the cable 5 is drawn so as to be bent on the cushioning member 135, the cable 5 can be constrained by the cushioning member 135 such that the cable 5 is not steeply bent toward the backside portion from the end portion, but the cable 5 is bent after the cable 5 is drawn upward from the end portion. Therefore, the degradation caused by the steep bending can be prevented at the base portion in the cable 5 having the characteristic that the core wire is easily disconnected by the metal fatigue due to the repeated bending. _

### Third Modification

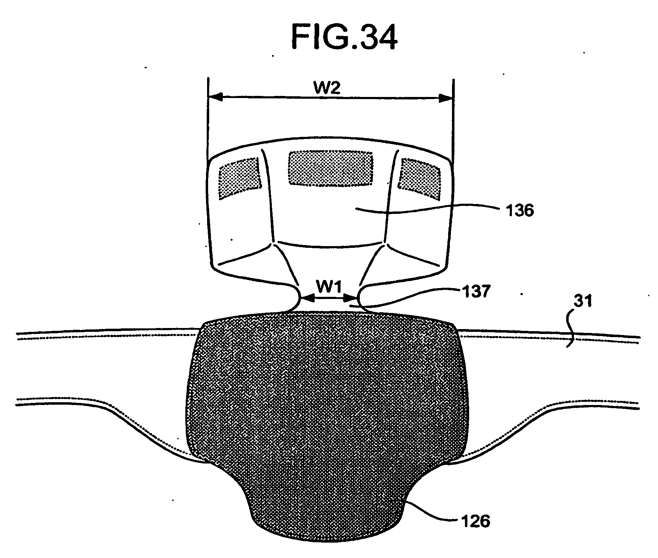

FIG. 34 is a partial rear view showing a configuration example of a receiver holder of a third modification. In the fifth embodiment, the flap 127 is formed such that the width W1 of the hinge pair 128 corresponds to the pouch width while the width W2 of the overlapping portion is widened. On the contrary, in the third modification, the flap 136 is formed so as to have the size and bending shape which exactly overlap the appearance shape of the pouch 122, and the width of the hinge pair 137 is decreased by a pinched shape. Therefore, the relationship of W1<W2 is relatively satisfied on the front-face projection.

### Seventh Embodiment

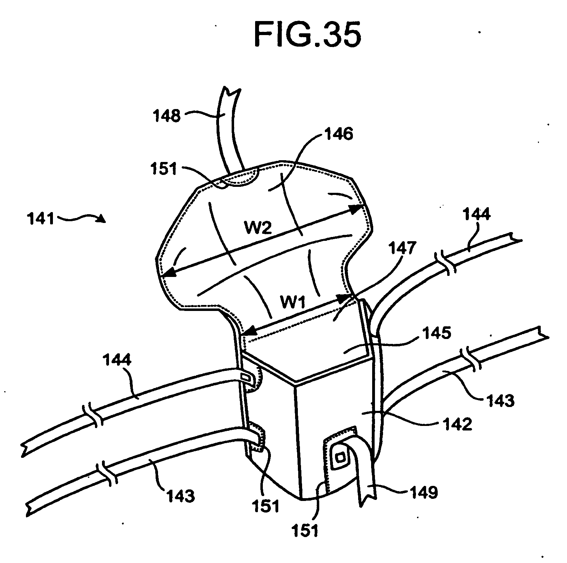

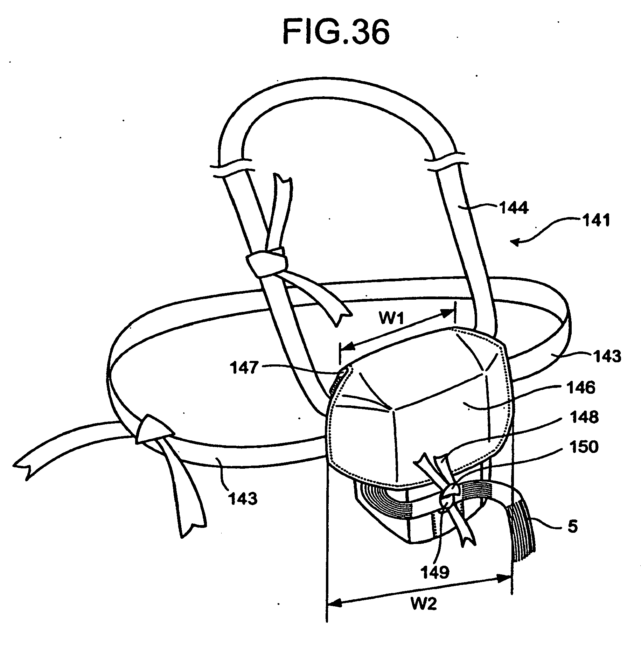

FIG. 35 is a perspective view showing a configuration example of a receiver holder according to a seventh embodiment, and FIG. 36 is a perspective view showing a state in which the flap is closed in the receiver holder of the seventh embodiment. The receiver holder 141 of the seventh embodiment is formed as the disposable type.

A receiver holder 141 of the seventh embodiment mainly includes a pouch 142, an abdomen flat string 143, and a shoulder flat string 144. The pouch 142, the abdomen flat string 143, and the shoulder flat string 144 are mainly formed by the nonwoven cloths which are suitable to the disposable use. The pouch 142 which is of the storage container is formed in the container shape by stitching the rims of the both sides and lower end along with the backing cloth portion and surface cloth portion using the nylon edge tape. As shown in FIG. 35, an opening 145 is provided on the upper end portion of the pouch 142 as the storage container, and the opening 145 is covered with a flap 146 as the cap member while freely opened and closed. The flap 146 is integrally formed by upwardly extending the upper end side of the backing cloth portion which forms the pouch 142, and the flap 146 has the structure in which the flap 146 is opened and closed with the fulcrum of a hinge pair 147 located on the backside while covering the opening 145 on the front-face side.

The flap 146 has the size and bending shape in which the front edge side overlaps the pouch fabric below the opening 145 in the outer surface on the front-face side of the pouch 142 when the opening 145 is closed. In the flap 146, a flap string 148 having the flat string configuration is stitched in the front-edge portion. The flap 146 is kept closed by connecting the flap string 148 to a flap string 149 stitched on the front-face lower portion side of the pouch 142. The flap strings 148 and 149 constitute a strap member 150. When the pouch 142 is placed on the body, the flap 146 is formed in the size and outline shape so as to hide the cable 5 drawn from the opening 145 to the backside through the right or left side of the hinge pair 147, in front-face projection in which the pouch 142 is viewed from the direction in front of the outer surface on the front-face side of the pouch 142. The flap 146 is formed such that the width W2 of the front-face side overlapping portion is larger than the width W1 of the hinge pair 147 on the backside. That is the width W2 on the front-face projection of the flap 146 is larger than the width of the pouch 142.

In the abdomen flat string 143 which is of the abdominal region placing member and the shoulder flat string 144 which is of the suspension placing member, each one end is fixed to an appropriate position on the right or left side of the pouch 142 by the stitching. The abdomen flat string 143 and the shoulder flat string 144 have the lengths enough to be suspended on the shoulder independently of the physical size. The abdomen flat strings 143 are tied together, and the shoulder flat strings 144 are tied together. Then, the excessive portion is cut and removed to freely adjust the length by the scissors. In the pouch 142 and the flap 146, the joint regions formed by stitching the abdomen flat string 143, the shoulder flat string 144, and flap strings 148 and 149 are reinforced by the synthetic leather 151.

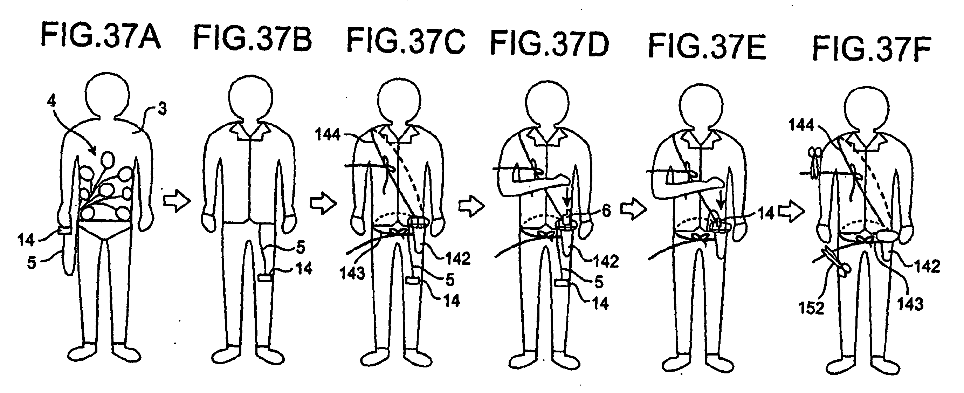

The procedure of placing the receiver holder 141 of the seventh embodiment on the subject 3 will be described below. FIGS. 37A to 37F are views sequentially showing the procedure of placing the receiver holder 141. As shown in FIG. 37A, the loop antennas 12a to 12h adhere to proper points in the body surface subject 3. This treatment is performed by the doctor. As shown in FIG. 37B, the subject 3 wears the cloths. At this point, the antenna jack unit 14 is in the standby state while hanging from the end of the cloths. As shown in FIG. 37C, the abdomen flat string 143 is wound around the abdomen circumference to tie the strings at a proper length position, the shoulder flat string 144 is suspended on the shoulder at a proper length position.

As shown in FIG. 37D, the receiver 6 whose antenna bay 17 is opened is stored in the pouch 142. The flap 146 is kept open. Then, as shown in FIG. 37E, the antenna jack unit 14 is attached to the antenna bay 17 of the receiver 6 stored in the pouch 142 while the cable 5 is passed through the outside (front-face side) of the abdomen flat string 143. The cable 5 is drawn from the opening 145 of the pouch 142 along the left side (or right side), and the flap 146 is closed by connecting the flap strings 148 and 149 as shown in FIG. 37F. At this point, as shown in FIG. 36, the path of the cable 5 which is drawn from the opening 145 and exposed outside the pouch 142 is constrained to the outer surface of the pouch 122 by the flap strings 148 and 149. Therefore, the looseness of the exposed cable 5 is prevented, and the unexpected external force is prevented from being applied to the cable 5, so that a lifetime of the cable 5 can be lengthened. Then, the lengths of the abdomen flat string 143 and shoulder flat string 144 are adjusted again, and the excessive portions are cut and removed by the scissors 152.

As described above, according to the seventy embodiment, the abdomen flat string 143 and shoulder flat string 144 having the sufficient lengths irrespective of the physical size are prepared, so that the placement state suitable to the physical size of the subject 3 can be secured by the method of simply cutting the excessive portion which is unique to the disposable type, and the length adjustment mechanism can be simplified. Although the pouch 142 is made of the inexpensive nonwoven cloth, the joint region with the abdomen flat string 143 or shoulder flat string 144 is reinforced by the synthetic leather 152, so that the stable placement state can be maintained. -

The invention is not limited to the above-described embodiments, but various modifications could be made without departing from the scope of the invention.

### INDUSTRIAL APPLICABILITY

The present invention is useful as the portable medical apparatus storage holder and the capsule endoscope medical care system in which the storage target is the portable medical apparatus connected to the detecting device placed on the subject body by the cable. Particularly the invention is suitable to the case where the storage target is the receiving apparatus for the capsule endoscope.

## Claims

1. A portable medical apparatus storage holder (7; 71; 91; 121; 141), comprising:
a storage container (41; 72; 92; 122; 142) in which a portable medical apparatus (6) is detachably stored from an opening, the portable medical apparatus (6) being placed on a subject body, and electrically connected to a detecting device (4) through a cable (5),
a placing member (31; 73; 143; 61; 74; 144) which detachably places the storage container on the subject body; and
a constraint member (57; 135) constraining a path of the cable (5) drawn from the opening (44) of the storage container in which the portable medical apparatus (6) is stored,
**characterized in that** the portable medical apparatus (6) is electrically connected to the detecting device (4) to record a predetermined electric displacement amount detected by the detecting device (4),
the constraint member (57; 135) is continuously connected to the storage container (41; 72; 92; 122; 142) or the placing member (31; 73; 143; 61; 74; 144),
the opening is an opening which is positioned so as to face the subject side while the storage container (41; 72; 92; 122; 142) is placed, the opening permitting the portable medical apparatus (6) to be inserted and detached, the opening permitting the cable (5) to be drawn,
the opening includes
a cap member (46; 76; 96; 127; 136; 146) which is continuously connected to the storage container (41; 72; 92; 122; 142) at a side on a top portion side of the opening by a hinge pair (47) to open and close the opening, the cap member (46) having a size in which an end side parallel to the hinge pair (47) overlaps an outer surface on a backside of the storage container (41; 72; 92; 122; 142) when the opening is closed; and
a joint structure (48) which joints an inner surface of the cap member (46; 76; 96; 127; 136; 146) and the outer surface on the backside of the storage container (41; 72; 92; 122; 142) in an overlapping range between the cap member (46; 76; 96; 127; 136; 146) and the storage container (41; 72; 92; 122; 142),
the cable (5) includes a plurality of cables, and
the constraint member (57; 135) has the joint structure (48), and constrains a path of a base portion of the cables by clamping a bundling member (13) with a joint structure portion, the bundling member (13) bundling the cables in a flat shape, the cables being drawn to the subject side from the opening of the storage container (41; 72; 92; 122; 142) in which the portable medical apparatus (6) is stored.

2. The portable medical apparatus storage holder according to claim 1, wherein the joint structure (48) is a joint structure (48) in which a surface fastener is used.

3. The portable medical apparatus storage holder according to claim 2, wherein the storage container (41; 72; 92; 122; 142) has a groove structure (56) in which the cables and the bundling member (13) are fitted in a vertical direction in the joint structure (48).

4. The portable medical apparatus storage holder according to any one of claims 1 to 3, wherein the storage container (41; 72; 92; 122, 142) includes a friction member (45; 59) in a backside thereof which is in contact with the subject body, the friction member (45; 59) having elasticity.

5. The portable medical apparatus storage holder according to any one of claims 1 to 3, wherein the storage container (41; 72; 92; 122; 142) and the cap member (46; 76; 96; 127; 136; 146) include friction members (45; 59) in backsides thereof which are in contact with the subject body, the friction members (45; 59) having elasticity.

6. The portable medical apparatus storage holder according to any one of claims 1 to 4, wherein the placing member (31; 73; 143; 61; 74; 144) includes an abdominal region placing member (31; 73; 143) and a suspension placing member (61; 74; 144), the abdominal region placing member (31; 73; 143) being rounded around an abdomen portion of the subject, the suspension placing member (61; 74; 144) being suspended on the shoulder of the subject while detachably attached at an arbitrary position of the abdominal region placing member (31; 73; 143).

7. The portable medical apparatus storage holder (7; 71; 91; 121; 141) according to any one of claims 1 to 6, wherein the placing member (31; 73; 143; 61; 74; 144) is freely adjustable in length according to a habitus of the subject.

8. The portable medical apparatus storage holder (7; 71; 91; 121; 141) according to claim 7, wherein the placing member (31; 73; 143; 61; 74; 144) is formed by a disposable material which can freely be cut with a cutting tool, and the placing member is freely adjustable in length by cutting the placing member according to the habitus of the subject.

9. The portable medical apparatus storage holder (7; 71; 91; 121; 141) according to claim 8, wherein the storage container (41; 72; 92; 122; 142) is formed by disposable nonwoven cloth, and a joint region with the placing member (31; 73; 143; 61; 74; 144) is reinforced by synthetic leather.

10. A capsule endoscope medical care system (1), comprising:
an capsule endoscope (2) which includes an imaging unit, an illumination unit for illuminating a region to be imaged, and a transmitting unit for transmitting image data obtained by the imaging unit to the outside, a subject being able to swallow the capsule endoscope;
a detecting device (4) which is placed in a subject body surface, the detecting device having an antenna structure (25) in which the image data transmitted from the transmitting unit is received as a predetermined electric displacement amount;
a portable medical apparatus (6) which is electrically connected to the detecting device (4) through a cable to record the image data received by the detecting device; and
a portable medical apparatus storage holder (7; 71; 91; 121; 141) according to any one of claims 1 to 9 which retains the portable medical apparatus (6) on a subject body.

## Patentansprüche

1. Aufnahmehalter (7; 71; 91; 121; 141) für eine tragbare medizinische Behandlungsvorrichtung, aufweisend:
ein Aufnahmebehältnis (41; 72; 92; 122; 142), in welchem eine tragbare medizinische Vorrichtung (6) aus einer Öffnung heraus entfernbar aufgenommen ist, wobei die tragbare medizinische Vorrichtung (6) an einem Körper einer Person angebracht wird und über ein Kabel (5) elektrisch mit einer Erkennungsvorrichtung (4) verbunden wird,
ein Anordnungsbauteil (31; 73; 143; 61; 74; 144), welches das Aufnahmebehältnis entfernbar an dem Körper einer Person anordnet; und
ein Einschränkungsbauteil (57; 135), das einen Pfad des Kabels (5), das aus der Öffnung (44) des Aufnahmebehältnisses herausgeführt ist, in welchem die tragbare medizinische Vorrichtung (6) aufbewahrt ist, einschränkt,
**dadurch gekennzeichnet, dass** die tragbare medizinische Vorrichtung (6) elektrisch mit der Erkennungsvorrichtung (4) verbunden ist, um einen bestimmten elektrischen Verschiebungsbetrag aufzuzeichnen, der von der Erkennungsvorrichtung (4) erkannt wird;
das Einschränkungsbauteil (57; 135) kontinuierlich mit dem Aufnahmebehältnis (41; 72; 92; 122; 142) oder dem Anordnungsbauteil (31; 73; 143; 61; 74; 144) verbunden ist;
die Öffnung eine Öffnung ist, die so angeordnet ist, dass sie zu der Seite der Person zeigt, wenn das Aufnahmebehältnis (41; 42; 92; 122; 142) an Ort und Stelle ist, wobei die Öffnung gestattet, dass die tragbare medizinische Vorrichtung (6) eingesetzt und entnommen wird und die Öffnung ein Herausführen des Kabels (5) gestattet, wobei die Öffnung aufweist:
ein Kappenbauteil (46; 76; 96; 127; 136; 146), welches kontinuierlich mit dem Aufnahmebehältnis (42; 72; 92; 122; 142) an einer Seite einer oberen Abschnittsseite der Öffnung durch ein Scharnierpaar (47) verbunden ist, um die Öffnung zu öffnen und zu verschließen, wobei das Kappenbauteil (46) eine Größe hat, bei der eine Endseite parallel zu dem Scharnierpaar (47) eine Außenfläche an einer Rückseite des Aufnahmebehältnisses (41; 72; 92; 122; 142) überlappt, wenn die Öffnung geschlossen ist; und
eine Verbindungsstruktur (48), welche eine Innenfläche des Kappenbauteils (46; 76; 96; 127; 136; 146) und die Außenfläche an der Rückseite des Aufnahmebehältnisses (41; 72; 92; 122; 142) in einem Überlappungsbereich zwischen dem Kappenbauteil (46; 76; 96; 127; 136; 146) und dem Aufnahmebehältnis (41; 72; 92; 122; 142) verbindet, wobei
das Kabel (5) eine Mehrzahl von Kabeln enthält und
das Einschränkungsbauteil (57; 135) die Verbindungsstruktur (48) aufweist und einen Pfad eines Basisabschnitts der Kabel einschränkt, indem ein Bündelungsbauteil (13) mit einem Verbindungsstrukturabschnitt geklemmt wird, wobei das Bündelungsbauteil (13) die Kabel in einer flachen Form zusammenbündelt, wobei die Kabel zur Seite der Person aus der Öffnung des Aufnahmebehältnisses (41; 72; 92; 122; 142), in welchem die tragbare medizinische Vorrichtung (6) aufgenommen ist, herausgeführt sind.

2. Aufnahmehalter für eine tragbare medizinische Vorrichtung nach Anspruch 1, wobei die Verbindungsstruktur (48) eine Verbindungsstruktur (48) ist, in welcher ein Flächenverschluss verwendet wird.

3. Aufnahmehalter für eine tragbare medizinische Vorrichtung nach Anspruch 2, wobei das Aufnahmebehältnis (41; 42; 92; 122; 142) eine Vertiefungsstruktur (56) hat, in welcher die Kabel und das Bündelungsbauteil (13) in einer vertikalen Richtung in der Verbindungsstruktur (48) eingesetzt sind.

4. Aufnahmehalter für eine tragbare medizinische Vorrichtung nach einem der Ansprüche 1 bis 3, wobei das Aufnahmebehältnis (41; 72; 92; 122; 142) ein Reibteil (45; 59) an einer Rückseite hiervon aufweist, welches in Kontakt mit dem Körper einer Person ist, wobei das Reibteil (45; 59) Elastizität hat.

5. Aufnahmehalter für eine tragbare medizinische Vorrichtung nach einem der Ansprüche 1 bis 3, wobei das Aufnahmebehältnis (41; 72; 92; 122; 142) und das Kappenbauteil (46; 76; 96; 127; 136; 146) in ihren Rückseiten Reibteile (45; 59) aufweisen, welche in Kontakt mit dem Körper einer Person sind, wobei die Reibteile (45; 59) Elastizität haben.

6. Aufnahmehalter für eine tragbare medizinische Vorrichtung nach einem der Ansprüche 1 bis 4, wobei das Anordnungsbauteil (31; 73; 143; 61; 74; 144) ein Anordnungsbauteil (31; 73; 143) für den abdominalen Bereich und ein Hängeanordnungsbauteil (61; 74; 144) aufweist, wobei das Anordnungsbauteil (31; 73; 143) für den abdominalen Bereich um einen abdominalen Bereich der Person geführt ist und das Aufhängeanordnungsbauteil (31; 73; 143) über die Schulter der Person gehängt ist, während es entfernbar an einer beliebigen Position des Anordnungsbauteils (31; 73; 143) für den abdominalen Bereich angebracht ist.

7. Aufnahmehalter (7; 71; 91; 121; 141) für eine tragbare medizinische Vorrichtung nach einem der Ansprüche 1 bis 6, wobei das Anordnungsbauteil (31; 73; 143; 61; 74; 144) abhängig von einer Statur der Person in seiner Länge frei einstellbar ist.

8. Aufnahmehalter (7; 71; 91; 121; 141) für eine tragbare medizinische Vorrichtung nach Anspruch 7, wobei das Anordnungsbauteil (31; 73; 143; 61; 74; 144) aus wegwerfbarem Material gebildet ist, welches durch ein Schneidwerkzeug beliebig schneidbar ist, wobei das Anordnungsbauteil frei in seiner Länge einstellbar ist, indem das Anordnungsbauteil gemäß der Statur der Person geschnitten wird.

9. Aufnahmehalter (7; 71; 91; 121; 141) für eine tragbare medizinische Vorrichtung nach Anspruch 8, wobei das Aufnahmebehältnis (41; 42; 92; 122; 142) aus einem wegwerfbaren Vlies ist und ein Verbindungsbereich mit dem Anordnungsbauteil (31; 73; 143; 61; 74; 144) durch Kunstleder verstärkt ist.

10. Medizinisches Behandlungssystem (1) mit einem Kapselendoskop, aufweisend:
ein Kapselendoskop (2), welches eine Abbildungseinheit, eine Beleuchtungseinheit zur Beleuchtung eines abzubildenden Bereichs und eine Übertragungseinheit aufweist zur Übertragung von Bilddaten, die von der Abbildungseinheit erhalten werden, zur Außenseite, wobei eine Person in der Lage ist, das Kapselendoskop zu schlucken;
eine Erkennungsvorrichtung (4), die an einer Oberfläche eines Körpers der Person angeordnet wird, wobei die Erkennungsvorrichtung eine Antennenstruktur (25) hat, in welcher die von der Übertragungseinheit übertragenen Bilddaten als ein bestimmter elektrischer Verschiebungsbetrag empfangen werden;
eine tragbare medizinische Vorrichtung (6), welche elektrisch mit der Erkennungsvorrichtung (4) über ein Kabel verbunden ist, um die von der Erkennungsvorrichtung empfangenen Daten aufzuzeichnen; und
einen Aufnahmehalter (7; 71; 91; 121; 141) für eine tragbare medizinische Vorrichtung nach einem der Ansprüche 1 bis 9, welcher die tragbare medizinische Vorrichtung (6) an einem Körper der Person hält.

## Revendications

1. Système de maintien d'appareil médical portable (7 ; 71 ; 91 ; 121 ; 141), comprenant :
un logement de maintien (41 ; 72 ; 92 ; 122 ; 142) dans lequel un appareil médical portable (6) est logé de manière détachable par une ouverture, l'appareil médical portable (6) étant placé sur le corps d'un sujet, et électriquement connecté à un dispositif de détection (4) par l'intermédiaire d'un câble (5),
un élément de mise en place (31 ; 73 ; 143 ; 61 ; 74 ; 144) qui met en place de manière détachable le logement de maintien sur le corps du sujet ; et
un élément de contrainte (57 ; 135) contraignant un chemin de passage du câble (5) tiré à partir de l'ouverture (44) du logement de maintien dans lequel l'appareil médical portable (6) est logé,
**caractérisé en ce que** l'appareil médical portable (6) est électriquement connecté au dispositif de détection (4) pour enregistrer une quantité prédéterminée de déplacement électrique détectée par le dispositif de détection (4),
l'élément de contrainte (57 ; 135) est connecté de manière continue au logement de maintien (41 ; 72 ; 92 ; 122 ; 142) ou à l'élément de mise en place (31 ; 73 ; 143 ; 61 ; 74 ; 144),
l'ouverture est une ouverture qui est positionnée de manière à faire face au côté du sujet lorsque le logement de maintien (41 ; 72 ; 92 ; 122 ; 142) est en place, l'ouverture permettant que l'appareil médical portable (6) soit inséré et détaché, l'ouverture permettant que le câble (5) soit tiré,
l'ouverture inclut
un élément de coiffe (46 ; 76 ; 96 ; 127 ; 136 ; 146) qui est connecté de manière continue au logement de maintien (41 ; 72 ; 92 ; 122 ; 142) au niveau d'un côté sur un côté de partie supérieure de l'ouverture par une paire de charnières (47) pour ouvrir et fermer l'ouverture, l'élément de coiffe (46) ayant une taille dans laquelle un côté d'extrémité parallèle à la paire de charnières (47) chevauche une surface extérieure sur un côté arrière du logement de maintien (41 ; 72 ; 92 ; 122 ; 142) lorsque l'ouverture est fermée ; et
une structure de jonction (48) qui joint une surface intérieure de l'élément de coiffe (46 ; 76 ; 96 ; 127 ; 136 ; 146) et la surface extérieure sur le côté arrière du logement de maintien (41 ; 72 ; 92 ; 122 ; 142) dans une plage de chevauchement entre l'élément de coiffe (46 ; 76 ; 96 ; 127 ; 136 ; 146) et le logement de maintien (41 ; 72 ; 92 ; 122 ; 142),
le câble (5) inclut une pluralité de câbles, et
l'élément de contrainte (57 ; 135) a la structure de jonction (48), et contraint un chemin de passage d'une partie de base des câbles en pinçant un élément de mise en faisceau (13) avec une partie de structure de jonction, l'élément de mise en faisceau (13) mettant les câbles en faisceau à une forme plate, les câbles étant tirés jusqu'au côté du sujet à partir de l'ouverture du logement de maintien (41 ; 72 ; 92 ; 122 ; 142) dans lequel l'appareil médical portable (6) est logé.

2. Système de maintien d'appareil médical portable selon la revendication 1, dans lequel la structure de jonction (48) est une structure de jonction (48) dans laquelle un dispositif de fixation de surface est utilisé.

3. Système de maintien d'appareil médical portable selon la revendication 2, dans lequel le logement de maintien (41 ; 72 ; 92 ; 122 ; 142) a une structure de rainure (56) dans laquelle les câbles et l'élément de mise en faisceau (13) sont disposés dans un sens vertical dans la structure de jonction (48).

4. Système de maintien d'appareil médical portable selon l'une quelconque des revendications 1 à 3, dans lequel le logement de maintien (41 ; 72 ; 92 ; 122 ; 142) inclut un élément de frottement (45 ; 59) dans un côté arrière de celui-ci qui est en contact avec le corps du sujet, l'élément de frottement (45 ; 59) ayant une élasticité.

5. Système de maintien d'appareil médical portable selon l'une quelconque des revendications 1 à 3, dans lequel le logement de maintien (41 ; 72 ; 92 ; 122 ; 142) et l'élément de coiffe (46 ; 76 ; 96 ; 127 ; 136 ; 146) incluent des éléments de frottement (45 ; 59) dans des côtés arrière de ceux-ci qui sont en contact avec le corps du sujet, les éléments de frottement (45 ; 59) ayant une élasticité.

6. Système de maintien d'appareil médical portable selon l'une quelconque des revendications 1 à 4, dans lequel l'élément de mise en place (31 ; 73 ; 143 ; 61 ; 74 ; 144) inclut un élément de mise en place de région abdominale (31 ; 73 ; 143) et un élément de mise en place en suspension (61 ; 74 ; 144), l'élément de mise en place de région abdominale (31 ; 73 ; 143) étant enroulé autour d'une partie d'abdomen du sujet, l'élément de mise en place en suspension (61 ; 74 ; 144) étant suspendu sur l'épaule du sujet tout en étant fixé de manière détachable en une position arbitraire de l'élément de mise en place de région abdominale (31 ; 73 ; 143).

7. Système de maintien d'appareil médical portable (7 ; 71 ; 91 ; 121 ; 141) selon l'une quelconque des revendications 1 à 6, dans lequel l'élément de mise en place (31 ; 73 ; 143 ; 61 ; 74 ; 144) est librement réglable en longueur en fonction d'un habitus du sujet.

8. Système de maintien d'appareil médical portable (7 ; 71 ; 91 ; 121 ; 141) selon la revendication 7, dans lequel l'élément de mise en place (31 ; 73 ; 143 ; 61 ; 74 ; 144) est formé d'un matériau jetable qui peut être librement coupé avec un outil de coupe, et l'élément de mise en place est librement réglable en longueur en coupant l'élément de mise en place en fonction de l'habitus du sujet.

9. Système de maintien d'appareil médical portable (7 ; 71 ; 91 ; 121 ; 141) selon la revendication 8, dans lequel le logement de maintien (41 ; 72 ; 92 ; 122 ; 142) est formé d'une étoffe non tissée jetable, et une région de jonction avec l'élément de mise en place (31 ; 73 ; 143 ; 61 ; 74 ; 144) est renforcée par du cuir synthétique.

10. Système de traitement médical (1) par endoscope à capsule, comprenant :
un endoscope à capsule (2) qui inclut une unité d'imagerie, une unité d'éclairage pour éclairer une région destinée à être imagée, et une unité de transmission pour transmettre des données d'image obtenues par l'unité d'imagerie vers l'extérieur, un sujet étant apte à avaler l'endoscope à capsule ;
un dispositif de détection (4) qui est placé sur une surface de corps d'un sujet, le dispositif de détection ayant une structure d'antenne (25) dans laquelle les données d'image transmises par l'unité de transmission sont reçues comme une quantité prédéterminée de déplacement électrique ;
un appareil médical portable (6) qui est électriquement connecté au dispositif de détection (4) par l'intermédiaire d'un câble pour enregistrer les données d'image reçues par le dispositif de détection ; et
un système de maintien d'appareil médical portable (7 ; 71 ; 91 ; 121 ; 141) selon l'une quelconque des revendications 1 à 9 qui maintient l'appareil médical portable (6) sur le corps d'un sujet.
